# EUROPEAN PATENT SPECIFICATION

(11) **EP 1 545 211 B1**
(45) Date of publication and mention of the grant of the patent: **30.04.2014**
(21) Application number: 03770513.4
(22) Date of filing: 29.09.2003
(51) Int. Cl.: A01N 43/90, A61K 31/475, C07D 405/06

(54) **NODULISPORIC ACID DERIVATIVE SPOT-ON FORMULATIONS FOR COMBATING PARASITES**
SPOT-ON FORMULIERUNGEN ZUR PARASITENBEKÄMPFUNG ENTHALTEND NODULISPORINSÄURE DERIVATEN
FORMULATIONS DE DERIVE D'ACIDE NODULISPORIQUE POUR UN TRAITEMENT CUTANE LOCALISE CONTRE LES PARASITES

(30) Priority: 02.10.2002 US 415627 P; 14.07.2003 US 618975
(43) Date of publication of application: 29.06.2005
(73) Proprietor: Merial Limited, Duluth, GA 30096 (US)
(72) Inventor: SOLL, Mark, D., Alpharetta, GA 30005 (US); BOECKH, Albert, Cumming, GA 30041 (US); DE BODE, Ronus, NL-5941 CV Velden (NL); VAN EIJK, Peter, Johannes, Sevaas, Savio, NL-6002 VD Weert (NL)
(74) Representative: Harding, Charles Thomas
(86) International application number: PCT/US2003/030500
(87) International publication number: WO 2004/030457

(56) References cited:
- EP-A- 0 249 409
- WO-A-96/29073
- WO-A-98/12196
- US-B1- 6 399 786
- MEINKE P T ET AL: "Chemical modification of nodulisporic acid A: preliminary structure-activity relationships" BIOORGANIC & MEDICINAL CHEMISTRY LETTERS, OXFORD, GB, vol. 10, no. 20, 16 October 2000 (2000-10-16), pages 2371-2374, XP004218029 ISSN: 0960-894X
- P. T. MEINKE, M. M. SMITH & W. L. SHOOP: "Nodulisporic acid: its chemistry and biology" CURR. TOPICS IN MEDIC. CHEM., vol. 2, no. 7, 2002, pages 655-674, XP009024399

## Description

### FIELD OF THE INVENTION

This invention relates to *inter alia* spot-on formulations for combating parasites in birds and mammals. I n p articular, this invention provides for spot-on formulations comprising a composition comprising at least one nodulisporic acid derivative and a pharmaceutically or veterinary acceptable liquid carrier vehicle. This invention also provides for an improved method for eradicating, controlling, and preventing parasite infestation in birds and mammals.

### BACKGROUND OF THE INVENTION

Animals such as mammals and birds are often susceptible to parasite infestations. These parasites may be ectoparasites, such as insects, and endoparasites such as filariae and worms.

Domesticated animal, such as cats and dogs, are often infested with one or more of the following ectoparasites:
- cat and dog fleas (Ctenocephalides felis, Ctenocephalides sp. and the like),
- ticks (Rhipicephalus sp., Ixodes sp., Dermacentor sp., Amblyomma sp. and the like), and
- mites (Demodex sp., Sarcoptes sp., Otodectes sp. and the like),
- lice (Trichodectes sp., Cheyletiella sp., Lignonathus sp., and the like), and
- flies (Hematobia sp., Musca sp., Stomoxys sp., Dermatobia sp., Coclyomia sp., and the like).

Fleas are a particular problem because not only do they adversely affect the health of the animal or human, but they also cause a great deal of psychological stress. Moreover, fleas are also vectors of pathogenic agents in animals, such as dog tapeworm (Dipylidium caninum), and humans.

Similarly, ticks are also harmful to the physical and psychological health of the animal or human. However, the most serious problem associated with ticks is that they are the vector of pathogenic agents, agents which cause diseases in both humans and animal. Major diseases which are caused by ticks include borreliosis (Lyme disease caused by Borrelia burgdorferi), babesiosis (or piroplasmosis caused by Babesia sp.) and rickettsiosis (also known as Rocky Mountain spotted fever). Ticks also release toxins which cause inflammation or paralysis in the host Occasionally, these toxins are fatal to the host, such as in the case of the Australian paralysis tick, Ixodes holocyclus.

Moreover, mites and lice are particularly difficult to combat since there are very few active substances which act on these parasites and they require frequent treatment.

Likewise, farm animals are also susceptible to parasite infestations. For example, cattle are affected by a large number of parasites. Likewise, arthropod pests, such as flea, lice and ticks, infest poultry. A parasite, which is very prevalent among farm animals, is a tick genus Boophilus, especially those of the species microplus (cattle tick), decoloratus and anulatus. Ticks, such as Boophilus microplus, a re p articularly difficult t o control because they live in the pasture where the farm animals graze. Other important parasites of cattle and sheep are listed as follows in order of decreasing importance:
- myiases such as Dermatobia hominis (known as Berne in Brazil) and Cochlyomia hominivorax (greenbottle); sheep myiases such as Lucilia sericata, L ucilia cuprina (known as blowfly strike in Australia, New Zealand and South Africa). These are flies whose larva constitutes the animal parasite;
- flies proper, namely those whose adult constitutes the parasite, such as Haematobia irritans (horn fly);
- lice such as Linognathus vitulorum, etc.; and
- mites such as Sarcoptes scabiei and Psoroptes ovis.
The above list is not exhaustive and other ectoparasites are well known in the art to be harmful to animals and humans. These include, for example migrating dipterous larvae.

Animals and humans also suffer from endoparasitical infections including, for example, helminthiasis, which is most frequently caused by a group of parasitic worms described as nematodes or roundworms. These parasites cause severe economic losses in pigs, sheep, horses, and cattle as well as affecting other domestic animals, such as dogs, cats and poultry. Other parasites which occur in the gastrointestinal tract of animals and humans include Ancylostoma, Anecator, Ascaris, Strongyloides, Trichinella, Capillaria, Toxocara, Toxascaris, Trichiris, Enterobius and parasites which are found in the blood or other tissues and organs such as filarial worms and the extra intestinal stages of Strogyloides, Toxocara and Trichinella.

Many insecticides exist in the art for treating parasites. These insecticides vary in their effectiveness to a particular parasite as well as their cost. However, the results of these insecticides is not always satisfactory because of, for example, the development of resistance by the parasite to the therapeutic agent, as is the case, for example, with carbamates, organophosphorus compounds and pyrethroids. Moreover, there is at the present time no truly effective method for controlling the set of parasites indicated above. Thus, there is a need in the art for more effective antiparasitic formulation treatment and protection of animal, *e.g*. mammals, fish and birds for a wide range of parasites. Moreover, there is a need in the art for an antiparasitic formulation which is easy to use on any type of domestic animal, irrespective of its size and the nature of its c oat and which do not need to be sprinkled over the entire body of the mammal, fish or bird. Further, the formulation should be effective for a long period of time thereby reducing the number of times it has to be applied.

Compounds, which exhibit a degree of activity against a wide range ectoparasites, are known in the art. The arylpyrazoles as a class are known in the art and are described, for example, in copending applications USSN 07/719,942; 08/933,016; 09/174,598; 08/863,182; and 08/863,692, as well as in U.S. Patent No. 5,576,429; U.S. Patent No. 5,122,530, EP-A-295,217, EP-A-352,944 and EP 295 177. Reference is made to, for example, U.S. patent No. 5,567,429, U.S. Patent No. 5,122,530, EP 295,117, and EP 846,686 A1 (or Banks GB 9,625,045, filed November 30, 1996 also believed to be equivalent to USSN 309,229, filed November 17, 1997). This class of insecticides is known to possess excellent activity against insects, such as ticks and fleas. Fipronil is a 1-N-aryl pyrazole that is particularly effective against fleas and ticks

Other compounds parasiticides that are known in the art to be effective are those which possess a macrocyclic lactone ring. These compounds are particularly effective against ectoparasites, including lice, blowflies, flies, mosquitoes, mites, migrating dipterous larvae, and ticks, as well as endoparasites, such as nematodes and roundworms. Compounds of this group include avermectins, milbemycins, and derivatives of these compounds, for example, ivermectin or emamectin. Such substances are described, for example, in U.S. Patents 3,950,360; 4,199,569; 4,879,749; and 5,268,710.

These compounds a re well known to a person skilled in the a rt and a re easily obtained either commercially or through techniques know in the art. Reference is made to the widely available technical and commercial literature. For avermectins, ivermectin and abamectin, reference may be made, for example, to the work "Ivermectin and Abamectin", 1989, by M.H. Fischer and H. Mrozik, William C. Campbell, published by Springer Verlag., or Albers-Schönberg et al. (1981), "Avermectins Structure Determination", J. Am. Chem. Soc., 103, 4216-4221. For doramectin, "Veterinary Parasitology", vol. 49, No. 1, July 1993, 5-15 may in particular be consulted. For milbemycins, reference may be made, *inter alia,* to Davies H.G. et al., 1986, "Avermectins and Milbemycins", Nat. Prod. Rep., 3, 87-121, Mrozik H. et al., 1983, Synthesis of Milbemycins from Avermectins , Tetrahedron Lett., 24, 5333-5336, U.S. Patent 4, 134, 973 and EP 677,054. These compounds are either natural products or are semi-synthetic derivatives thereof. The structure of these compounds is closely related, e.g., b y s haring a complex 1 6-member macrocyclic lactone ring. The natural product avermectins are disclosed in U.S. Patent 4,310,519 to Albers-Schönberg, et al., and the 22,23-dihydro avermectin compounds are disclosed in Chabala, et al., U.S. Patent No. 4,199,569. Mention is also made of Kitano, U.S. Patent No. 4,468,390, Beuvry et al., U.S. Patent No. 5,824,653, European Patent Application 0 007 812 A1, published June 2, 1980, U.K. Patent Specification 1 390 3 36, published April 9, 1975, European Patent Application 0 002 916 A2, and Ancare New Zealand Patent No. 237 086, *inter alia.* Naturally occurring milbemycins are described in Aoki et al., U.S. Patent No. 3,950,360 as well as in the various references cited in "The Merck Index" 12th ed., S. Budavari, Ed., Merck & Co., Inc. Whitehouse Station, New Jersey (1996). Semisynthetic derivatives of these classes of compounds are well known in the art and are described, for example, in U.S. Patent 5,077,308, U.S. Patent 4,859,657, U.S. Patent 4,963,582, U.S. Patent 4,855,317, U.S. Patent 4,871,719, U.S. Patent 4,874,749, U.S. Patent 4,427,663, U.S. Patent 4,310,519, U.S. Patent 4,199,569, U.S. Patent 5,055,596, U.S. Patent 4,973,711, U.S. Patent 4,978,677, U.S. Patent 4,920,148 and EP 667 054.
Other classes of compounds include insect growth regulating (IGR) compounds, which either mimic juvenile hormones or the inhibit synthesis of chintin. IGR compounds that mimic juvenile hormones include, for example, azadirachtin, diofenolan, fenoxycarb, hydroprene, kinoprene, methoprene, pyriproxyfen, tetrahydroazadirachtin, and 4-chloro-2-(2-chloro-2-methylpropyl)-5-(6-iodo-3-pyridylmethoxy)pyridizine-3(2H)-one. Chintin-synthesis inhibitors include, for example, chlorfluazuron, cyromazine, diflubenzuron, fluazuron, flucycloxuron, flufenoxuron, hexaflumuron, lufenuron, tebufenozide, teflubenzuron, triflumuron, 1-(2,6-difluorobenzoyl)-3-(2-fluoro-4-(trifluoromethyl)phenylurea,1-(2,6-difluorobenzoyl)-3-(2-fluoro-4-(1,1,2,2-tetrafluoroethoxy)phenylurea, and 1-(2,6-difluorobenzoyl)-3-(2-fluoro-4-trifluoromethyl) phenylurea.

Another class of compounds, which are known in the art as potent endo- and ectoantiparasitic agents, are nodulisporic acid derivatives. These compounds are based upon three structures, A, B or C, which have the following structures:
Nodulisporic acid (compound A) 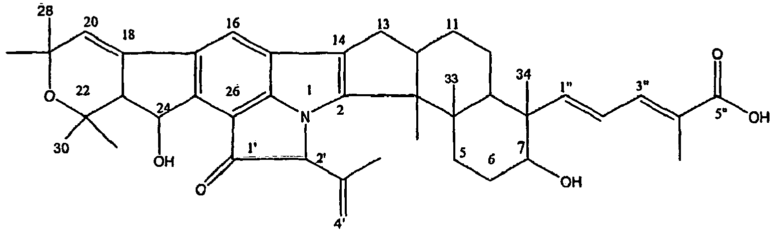
29,30-dihydro-20,30-oxa-nodulisporic acid (compound B) 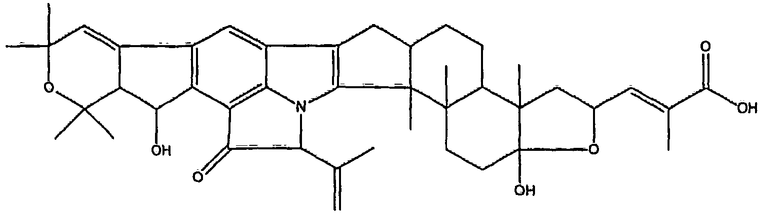 and
31-hydroxy-20,30-oxa-29,30,31,32-tetrahydro-nodulisporic acid (compound C) 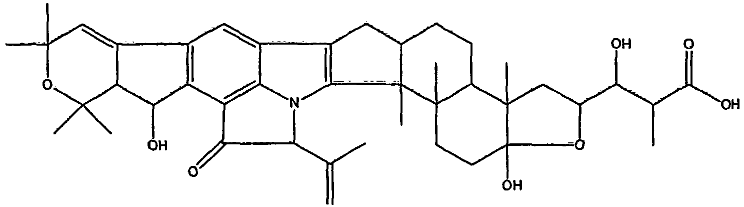
These compounds were obtained from the fermentation culture of *Nodulisporium sp.* MF-5954 (ATCC 74245) and the isolation and purification of the three nodulisporic acids are disclosed in US Patent 5,399,582. Derivatives of these compounds are described in WO 96/29073 and US Patent Nos. 5,945,317; 5,962,499; 5,834,260; 6,399,796; 6,221,894; 6,136,838; 5,595,991; and 5,614,546.

Nodulisporic acid derivatives possess potent activity against parasites, particularly helminths, ectoparasites, insects, and acarides, infecting man, animals and plants. These compounds have utility in human and animal health, agriculture and pest control in household and commercial areas.

The disease or group of diseases described generally as helminthiasis is due to infection of an animal host with parasitic worms known as helminths. Helminthiasis is a prevalent and serious economic problem in domesticated animals such as swine, sheep, horses, cattle, goats, dogs, cats, fish, buffalo, camels, Ilamas, reindeer, laboratory animals, furbearing animals, zoo animals and exotic species and poultry. Among the helminths, the group of worms described as nematodes causes widespread and often times serious infection in various species of animals. The most common genera of nematodes infecting the a nimals referred to above are Haemonchus, Trichostrongylus, Ostertagia, Nematodirus, Cooperia, Ascaris, Bunostomum, Oesophagostomum, Chabertia, Trichuris, Strongylus, Trichonema, Dictyocaulus, Capillaria, Habronema, Druschia, Heterakis, Toxocara, Ascaridia, Oxyuris, Ancylostoma, Uncinaria, Toxascaris and Parascaris. Certain of these, such as Nematodirus, Cooperia, and Oesophagostomum attack primarily the intestinal tract while others, such as Haemonchus and Ostertagia, are more prevalent in the stomach while still others such as Dictyocaulus are found in the lungs. Still other parasites may be located in other tissues and organs of the body such as the heart and blood vessels, subcutaneous and lymphatic tissue and the like. The parasitic infections The parasitic infections known as helminthiases lead to anemia, malnutrition, weakness, weight loss, severe damage to the walls of the intestinal tract and other tissues and organs and, if left untreated, may result in death of the infected host. The compounds of this invention have activity against these parasites, and in addition are also active against Dirofilaria in dogs and cats, Nematospiroides, Syphacia, Aspiculuris in rodents, arthropod ectoparasites of animals and birds such as ticks, mites such a s scabies lice, fleas, blowflies, a nd other biting insects in domesticated animals a nd poultry, such a s Tenophalides, Ixodes, Psoroptes, and Hemotobia, in sheep Lucilia sp., biting insects and such migrating dipterous larvae as Hypoderma sp. in cattle, Gasterophilus in horses, and Cuterebra sp. in rodents and nuisance flies including blood feeding flies and filth flies.

Nodulisporic acid derivatives are also useful against parasites which infect mammals, such as cats, dogs and humans. The most common genera of parasites of the gastro-intestinal tract of man are Ancylostoma, Necator, Ascaris, Strongyloides, Trichinella, Capillaria, Trichuris, and Enterobius. Other medically important genera of parasites which are found in the blood or other tissues and organs outside the gastrointestinal tract are the filiarial worms such as Wuchereria, Brugia, Onchocerca and Loa, Dracunuculus and extra intestinal stages of the intestinal worms Strongyloides and Trichinella. The compounds are also of value against arthropods parasitizing man, biting insects and other dipterous pests causing annoyance to man.

Nodulisporic acid derivatives are also active against household pests such as the cockroach, Blatella sp., clothes moth, Tineola sp., carpet beetle, Attagenus sp., the housefly Musca domestica as well as fleas, house dust mites, termites and ants.

nodulisporic acid derivatives are also useful against insect pests of stored grains such as Tribolium sp., Tenebrio sp. and of agricultural plants such as aphids, (Acyrthiosiphon sp.); against migratory orthopterans such as locusts and immature stages of insects living on plant tissue. The compounds are useful as a nematocide for the control of soil nematodes and plant parasites such as Meloidogyne sp., which may be of importance in agriculture. The compounds are also highly useful in treating acreage infested with fire and nests. The compounds are scattered above the infested area in low levels in bait formulations which are brought back to the nest. In addition to a direct-but-slow onset toxic effect on the fire ants, the compound has a long-term effect on the nest by sterilizing the queen which effectively destroys the nest

Nodulisporic acid and its derivatives are also effective against arthropod pests, for example fleas, ticks, ice a nd other biting insects in domesticated animals a nd poultry, such as Ctenophalides, Ixodes, Psoroptes, Lucilia and Hematobia.

It is known to combine the above-mentioned classes of compounds in order to achieve a broader spectrum of activity or, in some instances synergy. For example, U.S. Patent 5,945,317 discloses co-administering nodulisporic acid derivatives with avermectin or milbemycins, or other antihelmintic agents, such as morantel, pyrantel, or febantel, or benzimidizoles, such as thiabendazole or cambendazole. Other agents described therein include IGR compounds, such as lufenuron, or 1-N-arylpyrazoles, such a fipronil. See also, U.S. Patent 5,962,499 and 6,221,894. While it is known in the art that it is sometimes possible to combine various parasiticides in order to broaden the antiparasitical spectrum, it is not possible to predict, *a priori,* which combinations will work for a particular animal or disease state. For this reason, the results of various combinations is not always successful and there is a need in the art for more effective formulations which may be easily administered to the animal.

Various methods of formulating antiparasitical formulations are known in the art. These include oral formulations, baits, dietary supplements, powders, shampoos, etc. Formulations for localized topical applications of antiparasitical formulations are also known in the art. For example, pour-on solutions comprising 1-N-phenylpyrrazoles, such as fipronil, are known in the art and are described in copending application 08/933,016, herein incorporated by reference. Other methods for formulating antiparasitic agents include spot-on formulations.

Spot-on formulations are well known techniques for topically delivering an antiparasitic agent to a limited area of the host For example, U.S. Patent 5,045,536 describes such formulations for ectoparasites. Moreover, it is generally known in the art to formulate avermectin and milbemycin derivatives as spot-on formulations. See, e.g. U.S. Patent 5,045,536; EP 677,054; U.S. Patent 5,733,877; U.S. Patent 5,677,332; U.S. Patent 5,556,868; and U.S. Patent 5,723,488. U.S Patent Nos. 5,962,499 and 5,595,998 generally discusses formulating nodulisporic acid derivatives as pour-on or spot-on formulations, with or without additional antiparasitic agents. However, as discussed in U.S. Patent 5,045,536, a large number of solvent systems described in the art provide formulations for localized topical application which cause irritancy or toxicity to the host as well as being effective for a long period of time. Hence, there is a need in the art both for more effective for a longer period of time and less irritant or toxic formulations. Thus, there is a need in the art for a spot-on formulation, which is effective over a long period of time against a wide range of endoparasites and ectoparasites in birds and mammals.

### SUMMARY OF THE INVENTION

The invention provides *inter alia* for spot-on formulations for the treatment or prophylaxis of endoparasites of mammals, fish and birds, and in particular, cats, dogs, horses, chickens, sheep and cattle with the aim of ridding these hosts of all the parasites commonly encountered by birds and mammals. The invention also provides for effective and lasting destruction of ectoparasites, such as fleas, ticks, mites, *e.g.* itch mites, mosquitoes, flies and lice. Further, the spot-on formulations retain their efficacy over a long period of time, thereby reducing the number of applications of the formulation to the animal.

In particular this invention provides for

a spot-on formulation for the treatment or prophylaxis of parasite infestation in mammals or birds which comprises
(1) an effective amount of at least one nodulisporic acid derivative of the formula (I) 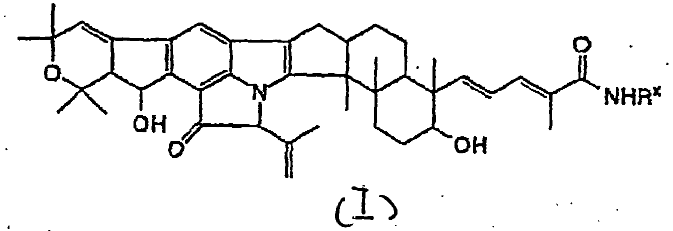 wherein R^{x} is selected from the group consisting of:
   H, CH₃, CH₂CH₃, C(CH₃)₃, CH₂CH₂CH₃, CH₂CH₂OH, CH(CO₂CH₃)CH₂OH, CH₂CO₂CH₃, CH₂CH(OCH₂CH₃)₂, CH₂CH₂OCH2CH₂OH, CH(CH₃)(CH₂)₃C(CH₃)₂OH, (CH₂)₃OH, (CH₂)₄OH, (CH₂)SOH, CH(CH₂OH)CH₂CH₃, NHC(CH₃)_{3,} CH₂CN, (CH₂)₆OH, CH₂CH(OH)CH₃, CH(CH₂OH)CH₂CH₂CH₃, CH₂CH₂SCH₃, CH₂CH₂SCH₂CH₃, CH₂CONH₂, CH(CH₃)(CH₂OH)₂, CH₂CH₂NHCH₂CH₂OH, CH(CH₂OH)(CH₂)₃CH₃, CH(CH₂OCH₃)CH₃, (CH₂)₂SH, (CH₂)₄NH₂, CH₂CH₂SO₂CH₃, CH₂CH₂S(O)CH₃, CH(CH(CH₃)₂)CH₂OH, (CH₂)₃NH₂, (CH₂)₃N(CH₂CH₃)₂, (CH₂)₃N(CH₃)₂, OCH₂CH₃, CH₂CH(OH)CH₂OH, OCH₃, CH₂CH₂OCH₃, CH₂CH₂NHC(O)CH₃, C(CH₃)₂CH₂OH, c-C₃H₅, cC₆H₁₁, (CH₂)₃OCH₂CH₃, CH₂CH=CH₂, C(CH₂CH₃)(CH₂OH)₂, CH₂C≡CH, CH₂CO₂CH₂CH₃, CH₂CH₂F, (CH₂)₃O(CH₂)₁₁CH₃, CH₂CH₂N(CH₃)₂, CH₂CH₂OCH₂CH₂NH₂, CH₂CF₃, NHCH₂CO₂CH₂CH₃, CH(CH₃)CO₂CH₃, C(CH₃)₂CH₂C(O)CH₃, CH(CO₂CH₂CH₃)₂, CH₂CH₃, CH(CH₂CH₂CH₃)CO₂CH₃, CH₂CH₂CH₂OCH₃, C(CH₃)₂CH₂O≡CH, (CH₂)₄CH₃, CH(CH₂CH₂CH₃)₂, (CH₂)SCH₃,CH₂CH₂CO₂H, CH(CH(CH₃)₂)CO₂CH₃, OCH₂CO₂H, CH(CH(CH₃)₂)CH₂OH, CH(CH(CH₃)₂)CH₂OH, CH(CH₃)CH₂OH, CH(CH₃)CH₂OH, CH(CH₃)₂, (CH₂)CH(CH₃)₂, CH(CH₃)CH₂CH₃, CH₂CH(CH₃)OH, (CH₂)₃CH₃, (CH₂)₂OCH₂CH₃, 1-adamantyl, (CH₂)₈CH₃, CH(CH₃)CH(CH₃)₂, (CH₂)₃NHCH₃, (CH₂)₂N(CH₂CH₃)₂, 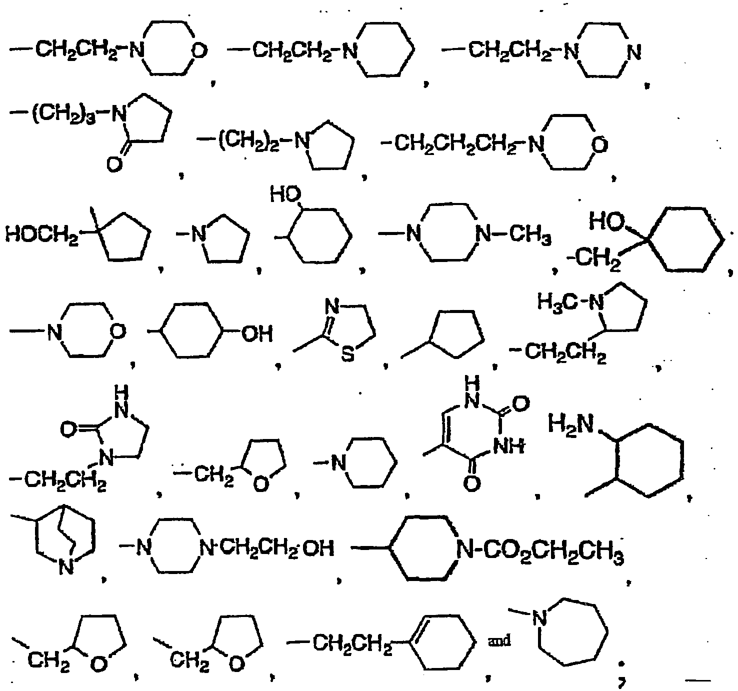
(2) a pharmaceutically or veterinary acceptable liquid carrier vehicle comprising a solvent and optionally a cosolvent wherein the solvent is selected from the group consisting of butyl diglycol, dipropylene glycol n-butyl ether, diethylene glycol monoethyl ether, ethylene glycol monomethyl ether, dipropylene glycol monomethyl ether, liquid polyoxyethylene glycols, propylene glycol, diethylene glycol monoethyl ether, ethylene glycol, and a mixture of at least two of these solvents and the cosolvent is selected from the group consisting of absolute ethanol, isopropanol or methanol; and (3) optionally, a crystallization inhibitor selected from the group consisting of an anionic surfactant, a cationic surfactant, a non-ionic surfactant, an amine salt, an amphoteric surfactant, polyvinylpyrrolidone, polyvinyl alcohols, copolymers of vinyl acetate and vinylpyrrolidone, polyethylene glycols, benzyl alcohol, mannitol, glycerol, sorbitol, polyoxyethylenated sorbitan esters; lecithin, sodium carboxymethylcellulose, and acrylic derivatives, or a mixture of these crystallization inhibitors.

Furthermore the invention provides for the spot-on formulations referred to above for use in treating or preventing parasite infestation in mammals, fish or birds.

This invention further provides for formulations which, when applied locally, will diffuse over the entire body of the host and then dry, without crystallizing, and which do not affect the appearance of the c oat after drying by, for example, leaving crystals or making the coat sticky. This has the further advantage in animals which groom themselves of not being orally ingested, where the therapeutic agent might not be well tolerated orally or might interact with other therapeutic agents.

The very high effectiveness of the method and of the formulations according to the invention provides not only for a high instantaneous effectiveness but also for an effectiveness of very long duration after the treatment of the animal.

This invention further provides for an amidation process to prepare amide derivatives of nodulisporic acid in higher yield with better purity. Novel intermediates in this process also form a part of this invention.

In this disclosure and in appended claims, terms such as "comprising" and "comprises" and the like, have the meanings ascribed to them in U.S. Patent Case Law. The terms "comprises" and "comprising" are open-ended and allow for the inclusion of additional ingredients or steps.

Clearly, a spot-on formulation spot-on comprising at least one nodulisporic acid derivative, advantageously t-butyl nodulisporamide in a veterinary acceptable liquid carrier vehicle is a basic or novel feature of the herein invention, as well as methods for preventing or treating parasites on an animal, e.g., dog, cat, by applying the formulation, e.g., monthly, and methods for preparing the formulations, e.g., by administering the ingredients, are also novel and basic features of the invention. That the invention performs as herein described is surprising, unexpected and nonobvious.

These and other embodiments are disclosed or are obvious from and encompassed by, the following Detailed Description.

### DETAILED DESCRIPTION

This invention provides for

a spot-on formulation for the treatment or prophylaxis of parasite infestation in mammals or birds which comprises
(1) an effective amount of at least one nodulisporic acid derivative of the formula (I) 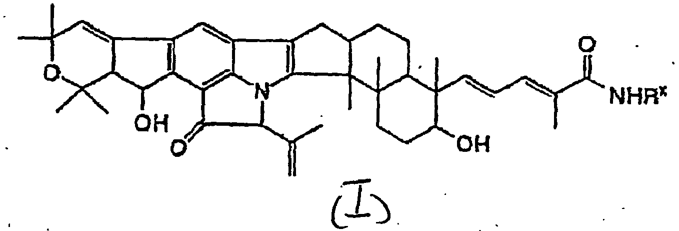 wherein R^{x} is selected from the group consisting of:
   H, CH₃, CH₂CH₃, C(CH₃)₃, CH₂CH₂CH₃, CH₂CH₂OH, CH(CO₂CH₃)CH₂OH, CH₂CO₂CH₃, CH₂CH(OCH₂CH₃)₂, CH₂CH₂OCH₂CH₂OH, CH(CH₃)(CH₂)₃C(CH₃)₂OH, (CH₂)₃OH, (CH₂)₄OH, (CH₂)SOH, CH(CH₂OH)CH₂CH₃, NHC(CH₃)₃, CH₂CN, (CH₂)₆OH, CH₂CH(OH)CH₃, CH(CH₂OH)CH₂CH₂CH₃, CH₂CH₂SCH₃, CH₂CH₂SCH₂CH₃, CH₂CONH₂, CH(CH₃)(CH₂OH)₂, CH₂CH₂NHCH₂CH₂OH, CH(CH₂OH)(CH₂)₃CH₃, CH(CH₂OCH₃)CH₃, (CH₂)₂SH, (CH₂)₄NH₂, CH₂CH₂SO₂CH₃, CH₂CH₂S(O)CH₃, CH(CH(CH₃)₂)CH₂OH, (CH₂)₃NH₂, (CH₂)₃N(CH₂CH₃)₂, (CH₂)₃N(CH₃)₂, OCH₂CH₃, CH₂CH(OH)CH₂OH, OCH₃, CH₂CH₂OCH₃, CH₂CH₂NHC(O)CH₃, C(CH₃)₂CH₂OH, c-C₃H₅, cC₆H₁₁, (CH₂)₃OCH₂CH₃, CH₂CH=CH₂, C(CH₂CH₃)(CH₂OH)₂, CH₂C≡CH, CH₂CO₂CH₂CH₃, CH₂CH₂F, (CH₂)₃O(CH₂)₁₁CH₃, CH₂CH₂N(CH₃)₂, CH₂CH₂OCH₂CH₂NH₂, CH₂CF₃, NHCH₂CO₂CH₂CH₃, CH(CH₃)CO₂CH₃, C(CH₃)₂CH₂C(O)CH₃, CH(CO₂CH₂CH₃)₂, CH₂CH₃, CH(CH₂CH₂CH₃)CO₂CH₃, CH₂CH₂CH₂OCH₃, C(CH₃)₂CH₂C≡CH, (CH₂)₄CH₃, CH(CH₂CH₂CH₃)₂, (CH₂)SCH₃,CH₂CH₂CO₂H, CH(CH(CH₃)₂)CO₂CH₃, OCH₂CO₂H, CH(CH(CH₃)₂)CH₂OH, CH(CH(CH₃)₂)CH₂OH, CH(CH₃)CH₂OH, CH(CH₃)CH₂OH, CH(CH₃)₂, (CH₂)CH(CH₃)₂, CH(CH₃)CH₂CH₃, CH₂CH(CH₃)OH, (CH₂)₃CH₃, (CH₂)₂OCH₂CH₃, 1-adamantyl, (CH₂)₈CH₃, CH(CH₃)CH(CH₃)₂, (CH₂)₃NHCH₃, (CH₂)₂N(CH₂CH₃)₂, 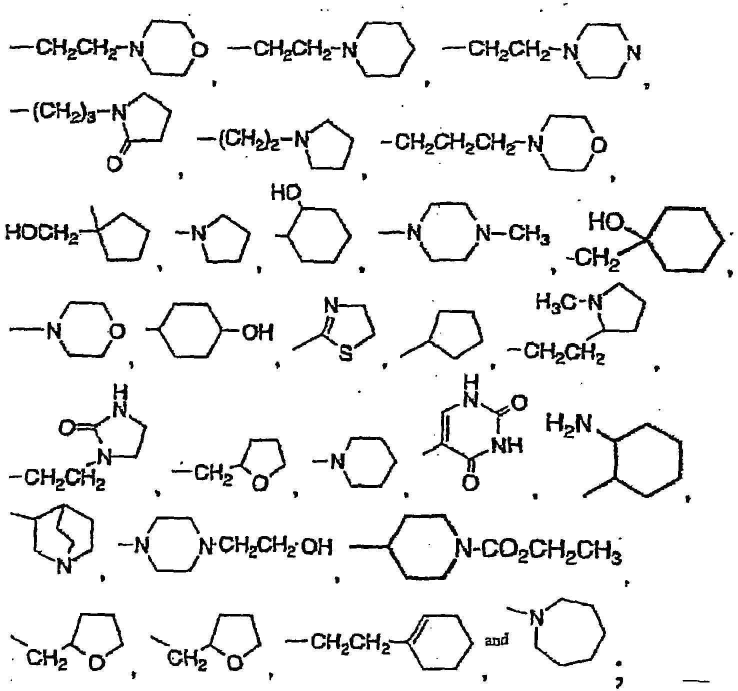
(2) a pharmaceutically or veterinary acceptable liquid carrier vehicle comprising a solvent and optionally a cosolvent wherein the solvent is selected from the group consisting of butyl diglycol, dipropylene glycol n-butyl ether, diethylene glycol monoethyl ether, ethylene glycol monoethyl either, dipropylene glycol monomethyl ether, liquid polyoxyethylene glycols, propylene glycol, diethylene glycol monoethyl ether, ethylene glycol, and a mixture of at least two of these solvents and the cosolvent is selected from the group consisting of absolute ethanol, isopropanol or methanol, and
(3) optionally, a crystallization inhibitor, selected from the group cansisting of an anionic surfactant, a cationic surfactant, a non-ionic surfactant, an amine salt, an amphoteric surfactant, polyvinylpyrrolidone, polyvinyl alcohols, copolymers of vinyl acetate and vinylpyrrolidone, polyethylene glycols, benzyl alcohol, mannitol, glycerol, sorbitol, polyoxyethylenated sorbitan esters; lecithin, sodium carboxymethylcellulose, and acrylic derivatives, or a mixture of these crystallization inhibitors.

An especially preferred nodulisporamide derivative is one wherein R^{x} is with t-butyl being most especially preferred.

"Alkyl" as well as other groups having the prefix "alk", such as alkoxy, alkanoyl, alkenyl, alkynyl and the like, means carbon chains which may be linear or branched or combinations thereof. Examples of alkyl groups include methyl, ethyl, propyl, isopropyl, butyl, sec- and tert-butyl, pentyl, hexyl, heptyl and the like. "Alkenyl", "alkynyl" and other like terms include carbon chains containing at least one unsaturated C-C bond.

The term "cycloalkyl" means carbocycles containing no heteroatoms, and includes mono-, bi- and tricyclic saturated carbocycles, as well as benzofused carbocycles. Examples of cycloalkyl include cyclopropyl, cyclobutyl, cyclopentyl, cyclohexyl, decahydronaphthalene, adamantane, indanyl, indenyl, fluorenyl, 1,2,3,4-tetrahydronaphalene and the like. Similarly, "cycloalkenyl" means carbocycles containing no heteroatoms and at least one non-aromatic C-C double bond, and include mono-, bi- and tricyclic partially saturated carbocycles, as well as benzofused cycloalkenes. Examples of cycloalkenyl include cyclohexenyl, indenyl, and the like.

The term "halogen" is intended to include the halogen atoms fluorine, chlorine, bromine and iodine.

The term "heterocycle", unless otherwise specfied, means mono- or bicyclic compounds that are saturated or partly unsaturated, as well as benzo- or heteroaromatic ring fused saturated heterocycles or partly unsaturated heterocycles, and containing from 1 to 4 heteroatoms independently selected from oxygen, sulfur and nitrogen. Examples of saturated heterocycles include morpholine, thiomorpholine, piperidine, piperazine, tetrahydropyran, tetrahydrofuran, dioxane, tetrahydrothiophene, oxazolidine, pyrrolidine; examples of partly unsaturated heterocycles include dihydropyran, dihydropyridazine, dihydrofuran, dihydrooxazole, dihydropyrazole, dihydropyridine, dihydropyridazine and the like. Examples of benzo- or heteroaromatic ring fused heterocycle include 2,3-dihydrobenzofuranyl, benzopyranyl, tetrahydroquinoline, tetrahydroisoquinoline, benzomorpholinyl, 1,4-benzodioxanyl, 2,3-dihydrofuro(2,3-b)pyridyl and the like.

The term "aryl" is intended to include mono- and bicyclic aromatic and heteroaromatic rings containing from 0 to 5 heteroatoms independently selected from nitrogen, oxygen and sulfur. The term "aryl" is also meant to include benzofused cycloalkyl, benzofused cycloalkenyl, and benzofused heterocyclic groups. Examples of "aryl" groups include phenyl, pyrrolyl, isoxazolyl, pyrazinyl, pyridinyl, oxazolyl, thiazolyl, imidazolyl, triazolyl, tetrazolyl, furanyl, triazinyl, thienyl, pyrimidinyl, pyridazinyl, pyrazinyl, naphthyl, benzoxazolyl, benzothiazolyl, benzimidazolyl, benzofuranyl, furo(2,3-B)pyridyl, 2,3dihydrofuro(2,3-b)pyridyl, benzoxazinyl, benzothiophenyl, quinolinyl, indolyl, 2,3-dihydrobenzofuranyl, benzopyranyl, 1,4-benzodioxanyl, indanyl, indenyl, fluorenyl, 1,2,3,4-tetrahydronaphthalene and the like.

Aroyl means arylcarbonyl in which aryl is as defined above.

Examples of NR^{c}R^{d} or NR^{g}R^{h} forming a 3- to 10-membered ring containing 0 to 2 additional heteroatoms selected from O, S(O)ₘ and N are aziridine, azetidine, pyrrolidine, piperidine, thiomorpholine, morpholine, piperazine, octahydroindole, tetrahydroisoquinoline and the like.

The term "optionally substituted" is intended to include both substituted and unsubstituted; thus, for example, optionally substituted aryl could represent a pentafluorophenyl or a phenyl ring.

Certain of the above defined terms may occur more than once in the above formula and upon such occurrence each term shall be defined independently of the other; thus, for example, OR^{a} at C4 may represent OH

Compounds of the invention are available commercially or can be prepared according to one or other of the processes or any other process coming within the competence of a person skilled in the art who is an expert in chemical synthesis. For the chemical preparation of the products of the invention, a person skilled in the art is regarded as having at his disposal, *inter alia,* the entire contents of "Chemical Abstracts" and of the documents which are cited therein. Semi-synthetic processes are described, for example, in U.S. Patent 6,399,786 or WO 96/29073.

A particularly effective synthetic method in order to prepare nodulisporamide compounds of the formula : 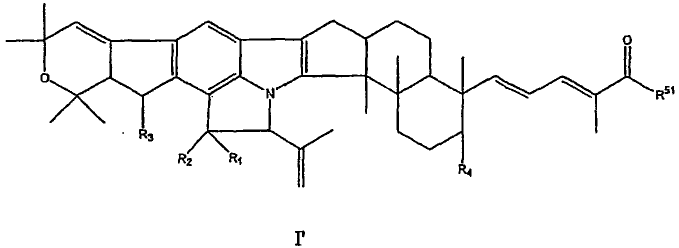 wherein R₁, R₂, R₃ and R₄ are defined above and R⁵¹ is NR^{c}R^{d} where R^{c}R^{d} are independently
(1) H,
(2) optionally substituted aryl,
(3) optionally substituted alkyl,
(4) optionally substituted alkenyl,
(5) optionally substituted alkynyl,
(6) optionally substituted cycloalkyl,
(7) optionally substituted cycloalkenyl, or
(8) optionally substituted heterocycle containing from 1 to 4 heteroatoms independently selected from oxygen, sulfur and nitrogen; where the substituents on the aryl, alkyl, alkenyl, cycloalkyl, cycloalkenyl, heterocycle, or alkynyl are from 1 to 10 groups independently selected from
   (i) hydroxy,
   (ii) alkyl,
   (iii) oxo,
   (iv) SO₂NR^{g}R^{h},
   (v) arylalkoxy,
   (vi) hydroxyalkyl,
   (vii) alkoxy,
   (viii) hydroxyalkoxy,
   (ix) aminoalkoxy,
   (x) cyano,
   (xi) mercapto,
   (xii) alkyl-S(O)ₘ,
   (xiii) cycloalkyl optionally substituted
      with 1 to 4 groups independently selected from R^{e},
   (xiv) cycloalkenyl,
   (xv) halogen,
   (xvi) alkanoyloxy,
   (xvii) C(O)NR^{g}R^{h},
   (xviii) CO₂Rⁱ,
   (xix) formyl,
   (xx) -NR^{g}R^{h},
   (xxi) 5 to 9-member heterocycle, which may be saturated or partially u nsaturated, c ontaining from 1 to 4 h eteroatoms independently selected from oxygen, sulfur and nitrogen, and optionally substituted with 1 to 5 groups independently selected from R^{e},
   (xxii) optionally substituted aryl, wherein the aryl substituents are 1,2-methylenedioxy or 1 to 5 groups independently selected from R^{e},
   (xxiii) optionally substituted arylalkoxy,
      wherein the aryl substituents are 1,2-methylenedioxy or 1 to 5 groups independently selected from R^{e},
   (xxiv) perfluoroalkyl; or
      - R^{c} and R^{d}: together with the N to which they are attached form a 3- to 10-member ring containing 0 to 2 additional heteroatoms selected from O, S(O)ₘ, and N, optionally substituted with 1 to 3 groups independently selected from R^{g}, hydroxy, thioxo and oxo;
      - R^{e} is: (1) halogen,
      (2) alkyl,
      (3) perfluoroalkyl,
      (4) "S(O)ₘRⁱ,
      (5) cyano,
      (6) nitro,
      (7) RⁱO(CH₂)ᵥ-,
      (8) RⁱCI₂(CH₂)ᵥ-,
      (9) RⁱOCO(CH₂)ᵥ-,
      (10) optionally substituted aryl where the substituents are from 1 to 3 of halogen, alkyl, alkoxy, or hydroxy,
      (11) SO₂NR^{g}R^{h}, or
      (12) amino;
      - R^{f} is: (1) alkyl,
      (2) X-alkyl, where X is O or S(O)ₘ,
      (3) alkenyl,
      (4) alkynyl,
      (5) perfluoroalkyl,
      (6) NY¹Y², where Y¹ and Y² are independently H or alkyl,
      (7) hydroxy,
      (8) halogen, and
      (9) alkanoyl amino,
      - R^{g} and R^{h}: are independently
      (1) hydrogen,
      (2) alkyl optionally substituted with hydroxy, amino, or CO₂Rⁱ
      (3) aryl optionally substituted with halogen, 1,2-methylenedioxy, alkoxy, alkyl or perfluoroalkyl,
      (4) arylalkyl, wherein the aryl is optionally substituted with perfluorolkyl or 1,2-methylenedioxy;
      (5) alkoxycarbonyl,
      (6) alkanoyl,
      (7) alkanoylalkyl,
      (9) arylalkoxycarbonyl,
      (10) aminocarbonyl,
      (11) monoalkylaminocarbonyl
      (12) dialkylaminocarbonyl; or
      - R^{g} and R^{h}: together with the N to which they are attached form a 3- to 7-member ring containing 0 to 2 additional heteroatoms selected from O, S(O)ₘ, and N, optionally substituted with 1 to 3 groups independently selected from R^{e} and oxo;
      - Rⁱ is: (1) hydrogen,
      (2) perfluoroalkyl,
      (3) alkyl,
      (4) optionally substituted aryl, or arylalkyl, where the aryl substituents are from 1 to 3 groups independently selected from halogen, alkyl, alkoxy, and hydroxy;
      - m is: 0 to 2; and
      - v is: 0 to 3; or
   said process comprising:
   (1) coupling a compound of formula II 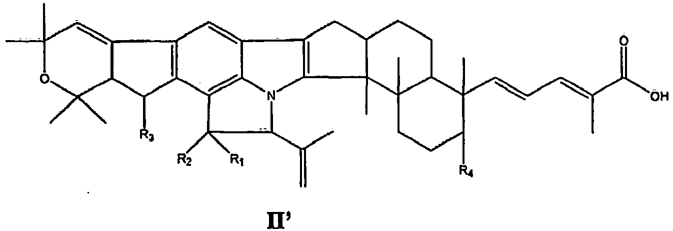 wherein
      R¹, R², R³, and R⁴ are defined above,
      with a compound of formula III: 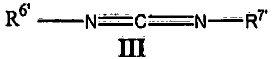
      wherein R^{6'} and R^{7'} can be independently selected from alkyl, aminoalkyl or cycloalkyl,
      in the presence of an organic solvent to produce a first intermediate compound of the formula: 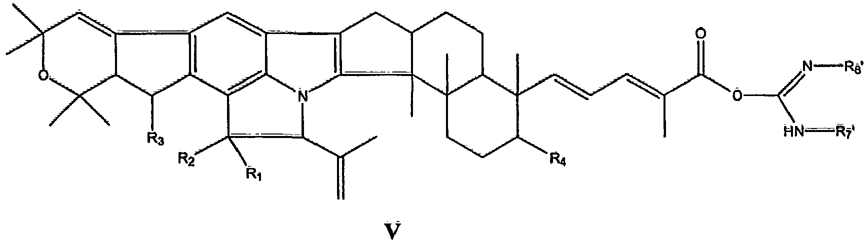
   (2) reacting the first intermediate compound with an activating compound ACT, such as a compound of formula IV: 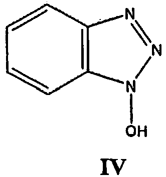 to produce a second intermediate compound of the formula VI': 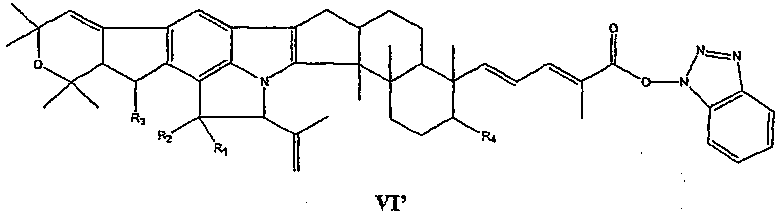
   (3) adding an amine of the formula HNR^{c}R^{d} to the second intermediate compound to obtain a compound of formula I'.

The advantage to the process is that the amidation reaction occurs under mild conditions, thereby reducing the potential of side reactions at the C₂₃-C₂₄ position or epimerization of C₇ of the starting material. This in turn increases the overall yield and purity of the final product. It is known that amidation under acidic conditions leads to dehydration of the C₂₃-C₂₄ position and amidation under basic conditions leads to epimerization of C₇; the inventive process is mild enough to reduce greatly the occurrence of these side reactions.

The process achieves these results by performing the amidation reaction via an active intermediate by reacting the nodulisporic acid compound with a compound of formula III. Preferred compounds of formula III are N-N'-Diisopropylcarbodiimide (DIPCDI) N-N'-dicyclohexylcarbodimide (DCC), and 1-[(3-dimethylamino)propyl]-3-ethylcarbodimide HCl salt (EDC) with DCC being especially preferred. This intermediate may be isolated or it may be reacted in one step with an activating compound such as a 1-hydroxybenzotriazole (HOBT) [Formula IV]. Other compounds which can be used as activating compounds include 2-hydroxypyridine-N-oxide (HOPO), 2-hydroxypyridine and 1-hydroxysuccinimide.

The amines of the formula HNR^{c}R^{d} are well known to a practioner of this art and are obtainable either commercially or by modification of known synthetic techniques, such as those found in "Organic Synthesis", a source that is well-known and used by a practitioner in the field. Preferred aminos include, for example, amines wherein R^{c} is H and R^{d} is selected from the group consisting of:
H, CH₃, CH₂CH₃, C(CH₃)₃, CH₂CH₂CH₃, CH₂CH₂OH, CH(CO₂CH₃)CH₂OH, CH₂CO₂CH₃, CH₂CH(OCH₂CH₃)₂, CH₂CH₂OCH₂CH₂OH, CH(CH₃)(CH₂)₃C(CH₃)₂OH, (CH₂)₃OH, (CH₂)₄OH, (CH₂)SOH, CH(CH₂OH)CH₂CH₃, NHC(CH₃)₃, CH₂CN, (CH₂)₆OH, CH₂CH(OH)CH₃, CH(CH₂OH)CH₂CH₂CH₃, CH₂CH₂SCH₃, CH₂CH₂SCH₂CH₃, CH₂CONH₂, CH(CH₃)(CH₂OH)₂, CH₂CH₂NHCH₂CH₂OH, CH(CH₂OH)(CH₂)₃CH₃, CH(CH₂OCH₃)CH₃, (CH₂)₂SH, (CH₂)₄NH₂, CH₂CH₂SO₂CH₃, CH₂CH₂S(O)CH₃, CH(CH(CH₃)₂)CH₂OH, (CH₂)₃NH₂, (CH₂)₃N(CH₂CH₃)₂, (CH₂)₃N(CH₃)₂, OCH₂CH₃, CH₂CH(OH)CH₂OH, OCH₃, CH₂CH₂OCH₃, CH₂CH₂NHC(O)CH₃, C(CH₃)₂CH₂OH, c-C₃H₅, cC₆H₁₁, (CH₂)₃OCH₂CH₃, CH₂CH=CH₂, C(CH₂CH₃)(CH₂OH)₂, CH₂C≡CH, CH₂CO₂CH₂CH₃, CH₂CH₂F, (CH₂)₃O(CH₂)₁₁CH₃, CH₂CH₂N(CH₃)₂, CH₂CH₂OCH₂CH₂NH₂, CH₂CF₃, NHCH₂CO₂CH₂CH₃, CH(CH₃)CO₂CH₃, C(CH₃)₂CH₂C(O)CH₃, CH(CO₂CH₂CH₃)₂, CH₂CH₃, CH(CH₂CH₂CH₃)CO₂CH₃, CH₂CH₂CH₂OCH₃, C(CH₃)₂CH₂C≡CH, (CH₂)₄CH₃, CH(CH₂CH₂CH₃)₂, (CH₂)SCH₃,CH₂CH₂CO₂H, CH(CH(CH₃)₂)CO₂CH₃, OCH₂CO₂H, CH(CH(CH₃)₂)CH₂OH, CH(CH(CH₃)₂)CH₂OH, CH(CH₃)CH₂OH, CH(CH₃)CH₂OH, CH(CH₃)₂, C(CH₃)₃, (CH₂)CH(CH₃)₂, CH(CH₃)CH₂CH₃, CH₂CH(CH₃)OH, (CH₂)₃CH₃, (CH₂)₂OCH₂CH₃, 1-adamantyl, (CH₂)₈CH₃, CH(CH₃)CH(CH₃)₂, (CH₂)₃NHCH₃, (CH₂)₂N(CH₂CH₃)₂,

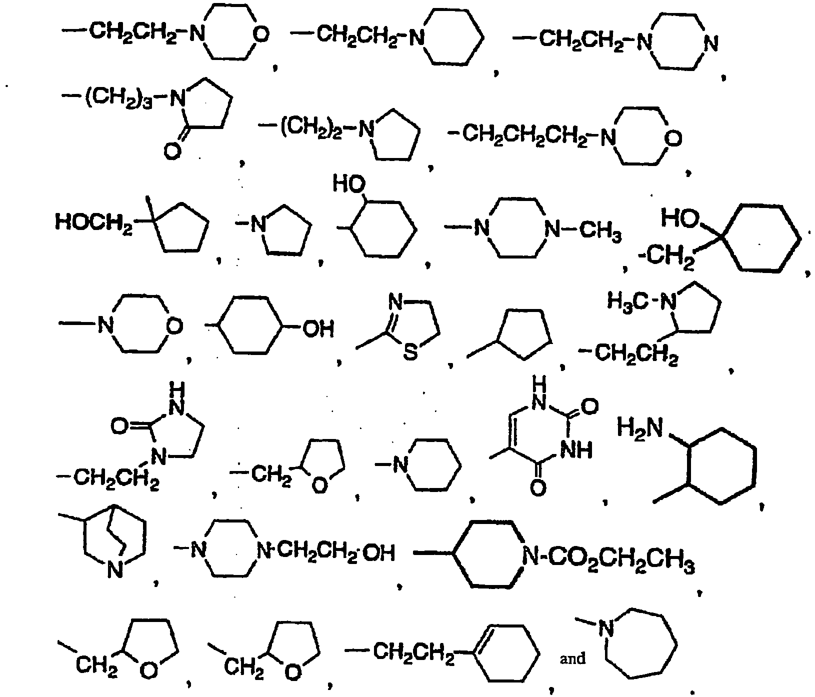

Preferred solvents for this reaction include halogenated hydrocarbons, such as dichloromethane and ethylene chloride, ethers, such as methyl t-butyl ether (MTBE), diethyl ether or tetraydrofuran (THF), or mixtures of the foregoing. Other solvents include polar aprotic solvents including but not limited to dimethoxymethane, 2-methyltetrahydrofuran, methyl iso-butylketone, benzotrifuoride and methylacetate. Perferably, the process uses a homogeneous solvent system that dissolves both the nodulisporic acid derivatives and the activation agents and avoids the prior process, which perform the activating/coupling reaction two phase water-organic solvent system, thereby permitting the reaction to run faster and obtain conversion more easily. A mixture of MTBE and THF is especially preferred. Preferred ranges of MTBE to THF are about 5:1 to about 1:2, with about 4:1 to about 2:3 being especially preferred.

The amount of DCC and HOBT should be in molar excess to achieve full conversion. A preferred range would be from about 1.1 to about 2.5, with about 1.2 to about 1.8 equivalents being especially preferred.

Reaction temperatures range from about 10°C to about 50°C with about 20°C to about 30°C being especially preferred.

Optionally, the nodulisporamide derivative may be recrystallized to obtain a product with better purity. Suitable recrystallization solvents include a mixture of a polar solvent such as water, acetonitrile, a cetone and an apolar solvent such as alkanes and cycloalkanes including but not limited to pentane, hexane, cyclohepatane, cyclohexane, cyclohepatane.
Preferred mixture of recrystallization solvent include acetone/heptane with few drops of water. An especially preferred mixture of solvents include first optionally recrystallizing from acetonitrile/water followed by acetone/heptane.

Administration of the inventive formulation may be intermittent in time and may be administered daily, weekly, biweekly, monthly, bimonthly, quarterly, or even for longer durations of time. The time period between treatments depends upon factors such as the parasite(s) being treated, the degree of infestation, the type of mammal or bird and the environment where it resides. It is well within the skill level of the practitioner to determine a specific administration period for a particular situation. This invention contemplates permanently combating a parasite in an environment in which the animal is subjected to strong parasitic pressure where the administration is at a frequency far below a daily administration in this case. For example, it is preferable for the treatment to be carried out monthly on dogs and on cats.

Spot-on formulations may be prepared by dissolving the active ingredients into the pharmaceutically or veterinary acceptable vehicle. Alternatively, the spot-on formulation can be prepared by encapsulation of the active ingredient to leave a residue of the therapeutic agent on the surface of the animal. These formulations will vary with regard to the weight of the therapeutic agent in the combination depending on the species of host animal to be treated, the severity and type of infection and the body weight of the host. The compounds may be administered continuously, particularly for prophylaxis, by known methods.

Generally, a dose of from about 0.001 to about 100 mg per kg of body weight, with a range of 0.25 to 50 mg/kg being especially preferred, given as a single dose or in divided doses for a period of from about 1 to about 60 days, preferably from about 1 to 30 days, will be satisfactory but, of course, there can be instance where higher or lower dosage ranges are indicated and such are within the scope of this invention. It is well within the routine skill of the practitioner to determine a particular dosing regimen for a specific host and parasite.

It also may be preferable to use controlled-release formulations.

The invention also relates to spot-on formulations referred to above for use in treating or preventing parasite infestation in mammals, fish or birds.

This invention also provides for formulations wherein the nodulisporic acid or derivative thereof is combined with a second active agent, such a parasiticide. Examples of classes of these compounds include avermectins, milbemycins, 1-N-arylpyrazoles, IGR compounds, etc., some of which are discussed above,

The formulations of the present invention may be for use by topical administration of a concentrated solution, suspension, microemulsion or emulsion for intermittent application to a spot on the animal, generally between the two shoulders. It has been discovered that the inventive formulations are especially active against parasites when the formulations are applied to mammals and birds, especially poultry, dogs, cats, sheep, pigs, cattle and horses. These formulations comprise a composition of an effective amount of at least one nodulisporic acid derivative dissolved in a pharmaceutical or veterinary acceptable carrier vehicle where a crystallization inhibitor is optionally present. The nodulisporic acid derivative are advantageously present in the inventive formulation in a proportion of about 1 to about 40%, preferably of about 1 to about 30% and most preferably about 5 to about 15% (percentages as weight by volume = W/V).

Also contemplated are the pharmaceutically or veterinary acceptable acid or base salts, where applicable, of the active compounds provided for herein. The term "acid" contemplates all pharmaceutically or veterinary acceptable inorganic or organic acids. Inorganic acids include mineral acids such as hydrohalic acids, such as hydrobromic and hydrochloric acids, sulfuric acids, phosphoric acids and nitric acids. Organic acids include all pharmaceutically or veterinary acceptable aliphatic, alicyclic and aromatic carboxylic acids, dicarboxylic acids tricarboxylic acids and fatty acids. Preferred acids are straight chain or branched, saturated or unsaturated C₁-C₂₀ aliphatic carboxylic acids, which are optionally substituted by halogen or by hydroxyl groups, or C₆-C₁₂ aromatic carboxylic acids. Examples of such acids are carbonic acid, formic acid, fumaric acid, acetic acid, propionic acid, isopropionic acid, valeric acid, α-hydroxy acids, such as glycolic acid and lactic acid, chloroacetic acid, benzoic acid, methane sulfonic acid, and salicylic acid. Examples of dicarboxylic acids include oxalic acid, malic acid, succinic acid, tartaric acid and maleic acid. An example of a tricarboxylic acid is citric acid. Fatty acids include all pharmaceutically or veterinary acceptable saturated or unsaturated aliphatic or aromatic carboxylic acids having 4 to 24 carbon atoms. Examples include butyric acid, isobutyric acid, sec-butyric acid, lauric acid, palmitic acid, stearic acid, oleic acid, linoleic a cid, linolenic a cid, and phenylsteric a cid. Other acids include gluconic acid, glycoheptonic acid and lactobionic acid.

The term "base" contemplates all pharmaceutically or veterinary acceptable inorganic or organic bases. Such bases include, for example, the alkali metal and alkaline earth metal salts, such as the lithium, sodium, potassium, magnesium or calcium salts. Organic bases include the common hydrocarbyl and heterocyclic amine salts, which include, for example, the morpholine and piperidine salts.

The organic solvent for the liquid carrier includes diethylene glycol monoethyl ether (Transcutol). These solvents can be supplemented by various excipients according to the nature of the desired phases, such as C₈-C₁₀ caprylic/capric triglyceride (Estasan or Miglyol 812), oleic acid or propylene glycol.

The liquid carrier may also comprise a microemulsion. Microemulsions are also well suited as the liquid carrier vehicle. Microemulsions are quaternary systems comprising an aqueous phase, an oily phase, a surfactant and a cosurfactant. They are translucent and isotropic liquids.

Microemulsions are composed of stable dispersions of microdroplets of the aqueous phase in the oily phase or conversely of microdroplets of the oily phase in the aqueous phase. The size of these microdroplets is less than 200 nm (1000 to 100,000 nm for emulsions). The interfacial film is composed of an alternation of surface-active (SA) and co-surface-active (Co-SA) molecules which, by lowering the interfacial tension, allows the microemulsion to be formed spontaneously.

The oily phase can in particular be formed from mineral or vegetable oils, from unsaturated polyglycosylated glycerides or from triglycerides; or alternatively from mixtures of such compounds. The oily phase preferably comprises triglycerides and more preferably medium-chain triglycerides, for example C₈-C₁₀ caprylic/capric triglyceride. The oily phase will represent, in particular, from about 2 to about 15%, more particularly from about 7 to about 10%, preferably from about 8 to about 9%, V/V of the microemulsion.

The aqueous phase includes, for example water or glycol derivatives, such as propylene glycol, glycol ethers, polyethylene glycols or glycerol. Propylene glycol, diethylene glycol monoethyl ether and dipropylene glycol monoethyl ether are especially preferred. Generally, the aqueous phase will represent a proportion from about 1 to about 4% V/V in the microemulsion.

Surfactants for the microemulsion include diethylene glycol monoethyl ether, dipropyelene glycol monomethyl ether, polyglycolysed C₈-C₁₀ glycerides or polyglyceryl-6 dioleate. In addition to these surfactants, the cosurfactants include short-chain alcohols, such as ethanol and propanol.

Some compounds are common to the three components discussed above, i.e., aqueous phase, surfactant and cosurfactant. However, it is well within the skill level of the practitioner to use different compounds for each component of the same formulation.

The cosurfactant to surfactant ratio will preferably be from about 1/7 to about 1/2. There will preferably be from about 25 to about 75% V/V of surfactant and from about 10 to about 55% V/V of cosurfactant in the microemulsion.

Likewise, the co-solvents are also well known to a practitioner in the formulation art. Preferred co-solvents are those which is a promoter of drying and include, for example, absolute ethanol, isopropanol (2-propanol) or methanol.

If present, it is preferred that the crystallization inhibitor is present in a proportion of about 1 to about 20% (W/V), preferably of about 5 to about 15%. The inhibitor preferably corresponds to the test in which 0.3 ml of a solution comprising 10% (W/V) of the compound of formula (I) in the liquid carrier and 10% of the inhibitor are deposited on a glass slide at 20°C and allowed to stand for 24 hours. The slide is then observed with the naked eye. Acceptable inhibitors are those whose addition provides for few or no crystals, and in particular less than 10 crystals, preferably 0 crystals.

Although this is not preferred, the formulation can optionally comprise water, in particular in a proportion of 0 to about 30% (volume by volume V/V), in particular of 0 to about 5%.

The formulation can also comprise an antioxidizing agent intended to inhibit oxidation in air, this agent being in particular present in a proportion of about 0.005 to about 1% (W/V), preferably of about 0.01 to about 0.05%.

Crystallization inhibitors which can be used in the invention include:
- polyvinylpyrrolidone, polyvinyl alcohols, copolymers of vinyl acetate and of vinylpyrrolidone, polyethylene glycols, benzyl alcohol, mannitol, glycerol, sorbitol or polyoxyethylenated esters of sorbitan; lecithin or sodium carboxymethylcellulose; or acrylic derivatives, such as methacrylates and others,
- anionic surfactants, such as alkaline stearates, in particular sodium, potassium or ammonium stearate; calcium stearate or triethanolamine stearate; sodium abietate; alkyl sulphates, in particular sodium lauryl sulphate and sodium cetyl sulphate; sodium dodecylbenzenesulphonate or sodium dioctyl sulphosuccinate; or fatty acids, in particular those derived from coconut oil,
- cationic surfactants, such as water-soluble quaternary ammonium salts of formula N⁺R'R"R"'R""Y⁻, in which the R radicals are identical or different optionally hydroxylated hydrocarbon radicals and Y⁻ is an anion of a strong acid, such as halide, sulphate and sulphonate anions; cetyltrimethylammonium bromide is one of the cationic surfactants which can be used,
- amine salts of formula N⁺R'R"R"', in which the R radicals are identical or different optionally hydroxylated hydrocarbon radicals; octadecylamine hydrochloride is one of the cationic surfactants which can be used,
- non-ionic surfactants, such as optionally polyoxyethylenated esters of sorbitan, in particular Polysorbate 80, or polyoxyethylenated alkyl ethers; polyethylene glycol stearate, polyoxyethylenated derivatives of castor oil, polyglycerol esters, polyoxyethylenated fatty alcohols, polyoxyethylenated fatty acids or copolymers of ethylene oxide and of propylene oxide,
- amphoteric surfactants, such as substituted lauryl compounds of betaine,
- or preferably a mixture of at least two of the compounds listed above.

In a particularly preferred embodiment, a crystallization inhibitor pair will be used. Such pairs include, for example, the combination of a film-forming agent of polymeric type and of a surface-active agent. These agents will be selected in particular from the compounds mentioned above as crystallization inhibitor.

Particularly preferred film-forming agents of polymeric type include:
- the various grades of polyvinylpyrrolidone,
- polyvinyl alcohols, and
- copolymers of vinyl acetate and of vinylpyrrolidone.

Especially preferred surface-active agents, include those made of non-ionic surfactants, preferably polyoxyethylenated esters of sorbitan and in particular the various grades of polysorbate, for example Polysorbate 80.

The film-forming agent and the surface-active agent can in particular be incorporated in similar or identical amounts within the limit of the total amounts of crystallization inhibitor mentioned elsewhere.

The pair thus constituted secures, in a noteworthy way, the objectives of absence of crystallization on the coat and of maintenance of the cosmetic appearance of the fur, that is to say without a tendency towards sticking or towards a sticky appearance, despite the high concentration of active material.

Particularly preferred antioxidizing agents are those conventional in the art and include, for example, butylated hydroxyanisole, butylated hydroxytoluene, ascorbic acid, sodium metabisulphite, propyl gallate, sodium thiosulphate or a mixture of not more than two of them.

The formulation adjuvants discussed above are well known to the practitioner in this art and may be obtained commercially or through known techniques. These concentrated compositions are generally prepared by simple mixing of the constituents as defined above; advantageously, the starting point is to mix the active material in the main solvent and then the other ingredients or adjuvants are added.

The volume applied can be of the order of about 0.3 to about 1 ml, preferably of the order of about 0.5 ml, for cats and of the order of about 0.3 to about 5 ml for dogs, depending on the weight of the animal.

The formulations according to the invention are extremely effective for long durations of time in the treatment of parasites such as fleas of mammals and, in particular, of small mammals such as dogs and cats. The inventive formulations exhibit a degree of effectiveness against other parasitic insects and in particular fleas and ticks. The inventive formulations further exhibit synergy when treating infestations cause by ectoparasites and endoparasites.

Other advantages and characteristics of the invention will become apparent on reading the following description, given by way of non-limiting examples.

### EXAMPLES

### EXAMPLE 1: Topical Spot-on Formulation (20% w/v t-butyl nodulisporamide)

A spot-on formulation comprising the following ingredients was prepared by mixing the following ingredients

| **Component** | **Amount (% w/v)** |
|---|---|
| t-butyl nodulisporamide | 20 |
| Tenox 2 (antioxidant) | 0.02 |
| Transcutol (carrier) | 100 qs |

### EXAMPLE 2: Topical Spot-on Formulation (10% w/v t-butyl nodulisporamide)

A spot-on formulation comprising t-butyl nodulisporamide was prepared by moving the following ingredients

| **Ingredient** | **Amount** (% w/v) |
|---|---|
| t-butyl nodulisporamide | 10 |
| Tenox 2 (antioxidant) | 0.02 |
| Miglyol 840 (additive) | 10 |
| Povidone (crystallization inhibitor) | 5 |
| Transcotol (liquid carrier) | 100 qs |

### EXAMPLE 3: Long Term Efficacy

The spot-on formulation according to Example 1 was applied to four dogs infested with fleas for a thirty-five day period. The results are summarized below

| | | | | | | |
|---|---|---|---|---|---|---|
| **Day** | **1** | **7** | **14** | **21** | **28** | **35** |
| **% effic** | **100** | **99.7** | **100** | **99.6** | **89.3** | **71.8** |

The data demonstrate that the spot-on formulation retained its efficacy for the thirty-five day period

### EXAMPLE 4: Long Term Efficacy

The spot-on formulation according to the first Example 1 (40 mg/kg) was applied to four cats infested with fleas and its efficacy for thirty-five days was measured. The results are summarized below

| | | | | | | |
|---|---|---|---|---|---|---|
| **Day** | **1** | **7** | **14** | **21** | **28** | **35** |
| **% effic** | **100** | **100** | **100** | **100** | **100** | **99.2** |

The data demonstrate that the spot-on formulation retained its efficacy for the thirty-five day period.

### EXAMPLE 5: Synthesis of t-butyl nodulisporamide

A reactor fitted with a stirrer was charged with 4.8 L of MTBE and 1.2 L of THF. Stirring was started and after mixing, 1.11 kg (1.5 mol) of nodulisporic acid A, used as a solvate prepared by crystallizing nodulisporic acid A from methanol and acetonitnte in a molar ratio of 1:1:1, was added to the to the reactor and allowed to dissolve Next, 0.26 kg HOBT.H₂O (1,70 mol) was charged to the reactor followed by the addition of 0.35 kg of a melt of DCC (1,70 mol) over a period of 1.5 h, keeping the temperature of the reaction between 20-30°C.

The mixture was then stirred for 4 h at 20-30°C. Following this, 0.27 kg of TBA (3. 69 mol) was added over a period of 1 h, again keeping the temperature of the reaction between 20-30°C. The mixture was then stirred for 1 h at 20-30°C.

Following stirring, the reaction mixture was filtered over filter cloth (Dralon) and the cake was washed with 4:1 (v/v) MTBE/THF (3 x 1.0 L) by means of a passive wash. The cake (DCU) is collected as solid organic waste (0.28 kg dry weight).

The filtrates were combined and washed sequentially with 5.0 L of 0.5 N aqueous hydrochloric acid and 5.0 L of aqueous saturated sodium bicarbonate. The washed filtrate (MTBE/THF) was concentrated *in vacuo* (30°C, 50-100 mBar) to ca. 6.0 L.

6.0 L of acetonitrile was added and the solution was concentrated *in vacuo* to 3.6 L. Water (2.4 L) was then added with stirring to the acetonitrile solution over a period of 2 h at 20-25°C. A yellow precipitate formed and this was filtered over filter cloth (Dralon) and then washed with acetonitrile/water (3:2 v/v) (2 x 1.5L). The solid was then air-dried for 3 h at ambient temperature and then *in vacuo* at 40-45° C to constant weight. The crude t-butyl nodulisporamide was subsequently recrystallized from acetone/heptane by first dissolving the crude t-butyl nodulisporamide in 2.5 L of acetone and then adding 5.8 L of heptane over a 2 h period of time with mechanical stirring at a temperature 20-25° C. The mixture was then aged for 2 h at 20° C.

The slurry was filtered over filter cloth and the cake sequentially washed with 1:4 (v/v) acetone/heptane (2 x 1.5L) and heptane (3.0L). The cake was air-dried for 3 h at ambient temperature and then under vacuum at 4 0-45° C t o constant weight t o give a molar yield of 75% of t-butyl nodulisporamide based upon nodulisporic acid A.

The above description of the invention is intended to be illustrative and not limiting. Various changes or modifications in the embodiments described herein may occur to those skilled in the art. These can be made without departing from the scope and spirit of the invention.

## Claims

1. A spot-on formulation for use in the treatment or prophylaxis of parasite infestation in mammals, fish or brids which comprises
(1) an effective amount of at least one nodulisporic acid derivative of the formula (I) 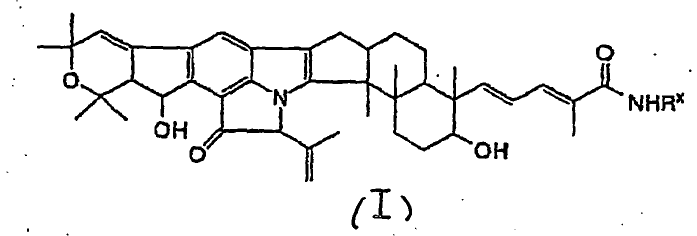 wherein R^{x} is selected from the group consisting of:
H, CH₃, CH₂CH₃, C(CH₃)₃, CH₂CH₂CH₃, CH₂CH₂OH, CH(CO₂CH₃)CH₂OH, CH₂CO₂CH₃, CH₂CH(OCH₂CH₃)₂, CH₂CH₂OCH₂CH₂OH, CH(CH₃)(CH₂)₃C(CH₃)₂OH, (CH₂)₃OH, (CH₂)₄OH, (CH₂)SOH, CH(CH₂OH)CH₂CH₃, NHC(CH₃)₃, CH₂CN, (CH₂)₆OH, CH₂CH(OH)CH₃, CH(CH₂OH)CH₂CH₂CH₃, CH₂CH₂SCH₃, CH₂CH₂SCH₂CH₃, CH₂CONH₂, CH(CH₃)(CH₂OH)₂, CH₂CH₂NHCH₂CH₂OH, CH(CH₂OH)(CH₂)₃CH₃, CH(CH₂OCH₃)CH₃, (CH₂)₂SH, (CH₂)₄NH₂, CH₂CH₂SO₂CH₃, CH₂CH₂S(O)CH₃, CH(CH(CH₃)₂)CH₂OH, (CH₂)₃NH₂, (CH₂)₃N(CH₂CH₃)₂, (CH₂)₃N(CH₃)₂, OCH₂CH₃, CH₂CH(OH)CH₂OH, OCH₃, CH₂CH₂OM₃, CH₂CH₂NH(O)CH₃, C(CH₃)₂CH₂OH, c-C₃H₅, cC₆H₁₁, (CH₂)₃OCH₂CH₃, CH₂CH=CH₂, C(CH₂CH₃)(CH₂OH)₂, CH₂C≡CH, CH₂CO₂CH₂CH₃, CH₂CH₂F, (CH₂)₃O(CH₂)₁₁CH₃, CH₂CH₇N(CH₃)₂, CH₂CH₂OCH₂CH₂NH₂, CH₂CF₃, NHCH₂CO₂CH₂CH₃, CH(CH₃)CO₂CH₃, C(CH₃)₂CH₇C(O)CH₃, CH(CO₂CH₂CH₃)₂, CH₂CH₃, CH(CH₂CH₇CH₃)CO₂CH₃, CH₂CH₂CH₂OCH₃, C(CH₃)₂CH₂OC≡CH, (CH₂)₄CH₃, CH(CH₂CH₂CH₃)₂, (CH₂)SCH₃,CH₂CH₂CO₂H, CH(CH(CH₃)₂)CO₂CH₃, OCH₂CO₂H, CH(CH(CH₃)₂)CH₂OH, CH(CH(CH₃)₂)CH₂OH, CH(CH₃)CH₂OH, CH(CH₃)CH₂OH, CH(CH₃)₂, (CH₂)CH(CH₃)₂, CH(CH₃)CH₂CS₃, CH₂CH(CH₃)OH, (CH₂)₃CH₃, (CH₂)₂OCH₂CH₃, 1-adamanlyl, (CH₂)₈CH₃, CH(CH₃)CH(CH₃)₂, (CH₂)₃NHCH₃, (CH₂)₂N(CH₂CH₃)₂, 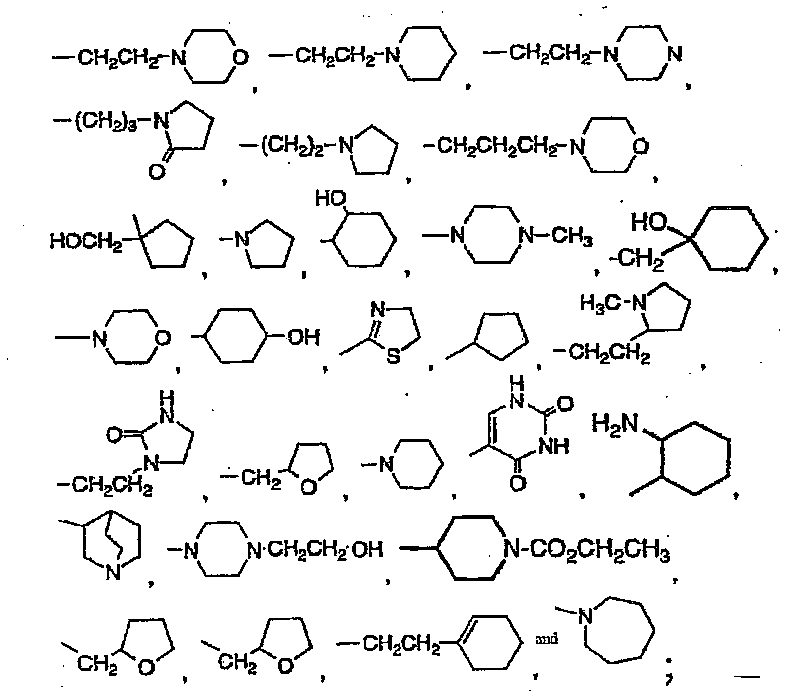
(2) a pharmaceutically or veterinary acceptable liquid carrier vehicle comprising a solvent and optionally a cosolvent wherein the solvent is selected from the group consisting of butyl diglycol, dipropylene glycol n-butyl ether, diethylene glycol monoetlryl ether, ethylene glycol monomethyl ether, dipropylene glycol monomethyl ether, liquid polyoxyethylene glycols, propylene glycol, diethylene glycol monoethyl ether, ethylene glycol, and a mixture of at least two of these solvents and the cosolvent is selected from the group consisting of absolute ethanol, isopropanol or methanol; and
(3) optionally, a crystallization inhibitor selected from the group consisting of an anionic surfactant, a cationic surfactant, a non-ionic surfacant, an amine salt, an amphoteric surfactant, polyvinylpyrrolidone, polyvinyl alcohols, copolymers of vinyl acetate and vinylpyrrolidone, polyethylene glycols, benzyl alcohol, mannitol, glycerol, sorbitol, p olyoxyethylenated sorbitan esters; lecithin, sodium carboxymethylcellulose, and acrylic derivatives, or a mixture of these crystallization inhibitors.

2. The spot-on formulation according to claim 1, wherein R^{x} is C(CH₃)₃.

3. The spot-on formulation according to claim 1, wherein the liquid carrier vehicle comprises a microemulsion.

4. The spot-on formulation according to claim 1, wherein the liquid carrier vehicle further comprises an excipient.

5. The spot-on formulation according to claim 4, wherein the excipient is C₈-C₁₀ caprylic/capric triglycerides, oleic acid or propylene glycol.

6. The spot-on formulation according to claim 5, wherein the spot-on formulation further comprises an antioxidant.

7. The spot-on formulation according to claim 6, wherein the antioxidant is selected from the group consisting of butylated hydroxyanisole, butylated hydroxytoluene, ascorbic acid, sodium metabisulphite, propyl gallate, and sodium thiosulphate.

8. The spot-on formulation according to claim 7, wherein the compound of formula (I) is t-butyl nodulisporamide, the carrier medium comprises diethylene glycol monoethyl ether and C₈-C₁₀ caprylic/capric triglycerides, and the antioxidant is butylated hydroxytoluene.

9. The spot-on formulation according to claim 1, wherein the combination comprises about 0.001 to about 100 mg/kg of weight of mammal or bird of a compound of formula (I).

10. The spot-on formulation according to claim 2, wherein the combination comprises about 1 to about 5 0 m g/kg of weight of mammal or bird of a compound of formula (I).

11. The spot-on formulation according to claim 1, which comprises crystallization inhibitor and further comprises an antioxidant.

12. The spot-on formulation according to claim 11, wherein about 0.005 to about 1% (W/V) of antioxidant is present and the antioxidant is selected from the group consisting of butylated hydroxyanisole, butylated hydroxytoluene, ascorbic acid, sodium metabisulphite, propyl gallate, and sodium thiosulphate.

13. The spot-on formulation according to claim 12, wherein the crystallization inhibitor is present in an amount from about 1 to about 20% W/V.

14. The spot-on formulation according to claim 13, wherein
- the anionic surfactant is alkaline stearates, sodium abietate; alkyl sulphates; sodium dodecylbenzenesulphonate, sodium dioctylsulphosuccinate; and fatty acids;
- the cationic surfactant is water-soluble quaternary ammonium salts of formula N⁺R'R"R' "R" " Y⁻ in which the radicals R independently are hydrocarbon radicals, optionally hydroxylated, and Y⁻ is an anion of a strong acid;
- the amine salt is an amine salt of N⁺R'R"R' " in which the radicals R independently are optionally hydroxylated hydrocarbon radicals;
- the non-ionic surfactant is optionally polyoxyethylenated sorbitan esters, polyoxyethylenated alkyl ethers; polyethylene glycol stearate, polyoxyethylenated derivatives of castor oil, polyglycerol esters, polyoxyethylenated fatty alcohols, polyoxyethylenated fatty acids, copolymers of ethylene oxide and propylene oxide; and
- the amphoteric surfactant is lauryl-substituted betaine compounds.

15. The spot-on formulation according to claim 13, where the crystallization inhibitor is a crystallization inhibitor system comprising a polymeric film-forming agent and a surfactant.

16. The spot-on formulation according to claim 15, wherein the polymeric film-forming agent is polyvinylpyrrolidone, polyvinyl alcohols, or a copolymer of vinyl acetate and polyvinylpyrrolidone and the surfactant is a non-ionic surfactant.

17. The spot-on formulation according to claim 16, wherein the crystallization inhibitor system is a mixture of polyvinylpyrrolidone and polyoxethylene (20) sorbitan mono-oleate.

18. The spot-on formulation according to claim 12, wherein the compound of formula (I) is t-butyl nodulisporamide, the liquid carrier vehicle is diethylene glycol monoethyl ether, the crystallization inhibitor is pyrrolidone and the antioxidant is butylated hydroxytoluene.

19. A spot-on formulation for use according to claim 1, wherein the parasite is an ectoparasite.

20. A spot-on formulation for use according to claim 1, wherein the parasite is an endoparasite.

21. A spot-on formulation for use according to claim 1, wherein the mammal is a cat, dog, horse, cattle or sheep.

22. A spot-on formulation for use according to claim 21, wherein the parasite is a flea or tick.

23. A spot-on formulation for use according to claim 1, wherein the mammal is a human.

24. A spot-on formulation for use according to claim 19, wherein the ectoparasites are mites, ticks, mosquitoes, flies or a combination of the foregoing.

25. The spot-on formulation for use according to claim 1, wherein the administration is bimonthly.

26. The spot-on formulation for use according to claim 1, wherein the administration is quarterly.

27. The spot-on formulation for use according to claim 1, wherein the administration is monthly.

28. A spot-on formulation for use according to claim 1 which is suitable for localized cutaneous application to a mammal with absorption and a resultant plasma concentration of the compound(s) of formula (I) wherein the liquid carrier vehicle comprises diethylene glycol monoethyl ether, and at least one antioxidant.

29. A spot-on formulation for use according to claim 1, wherein the mammal is a cat or dog and wherein said formulation is suitable for localized cutaneous application to the cat or dog, between the shoulders, at a frequency not greater than monthly, wherein the formulation contains an organic solvent, an antioxidant and/or a crystallization inhibitor wherein:
the organic solvent comprises diethyl glycol monoethyl ether; said solvent optionally supplemented by C₈-C₁₀ caprylic/capric triglyceride, oleic acid or propylene glycol;
the antioxidant is selected from the group consisting of butylated hydroxyanisole, butylated hydroxytoluene, ascorbic acid, sodium metabisuphite, propylgallate, and sodium theosulphate; and
the crystallization inhibitor selected from the group consisting of polyvinylpyrrolidone, copolymers of vinyl acetate and vinylpyrrolidone, polyoxyethylenated sorbitan esters and mixtures thereof;
whereby there is a prolonged release of formula (I) in or on the body of the cat or dog.

## Patentansprüche

1. Eine Spot-on-Formulierung zur Verwendung bei der Behandlung oder Prophylaxe eines Parasitenbefalls bei Säugern, Fischen oder Vögeln, welche umfasst
(1) eine wirksame Menge mindestens eines Nodulisporsäurederivats der Formel (I) 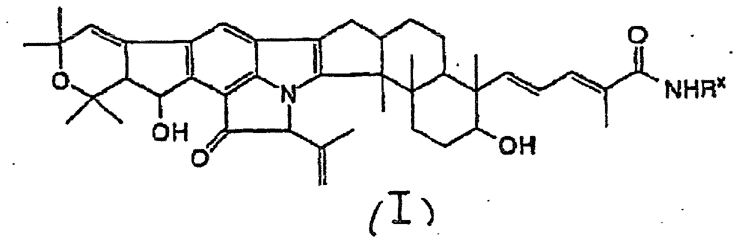 wobei R^{x} ausgewählt ist aus der Gruppe bestehend aus:
H, CH₃, CH₂CH₃, C(CH₃)₃, CH₂CH₂CH₃, CH₂CH₂OH, CH(CO₂CH₃)CH₂OH, CH₂CO₂CH₃, CH₂CH(OCH₂CH₃)₂, CH₂CH₂OCH₂CH₂OH, CH(CH₃)(CR₂)₃C(CH₃)₂OH, (CH₂)₃OH, (CH₂)₄OH, (CH₂)SOH, CH(CH₂OH)CH₂CH₃], NHC(CH₃)₃, CH₂CN, (CH₂)₆OH, CH₂CH(OH)CH₃, CH(CH₂OH)CH₂CH₂CH₂CH₃, CH₂CH₂SCH₃, CH₂CH₂SCH₂CH₃, CH₂CONH₂, CH(CH₃)(CH₂OH)₂, CH₂CH₂NHCH₂CH₂OH, CH(CH₂OH)(CH₂)₃CH₃, CH(CH₂OCH₃)CH₃, (CH₂)₂SH, (CH₂)₄NH₂, CH₂CH₂SO₂CH₃, CH₂CH₂S(O)CH₃, CH(CH(CH₃)₂)CH₂OH, (CH₂)₃NH₂, (CH₂)₃N(CH₂CH₃)₂, (CH₂)₃N(CH₃)₂, OCH₂CH₃, CH₂CH(OH)CH₂OH, OCH₃, CH₂CH₂OCH₃, CH₂CH₂NHC(O)CH₃, C(CH₃)₂CH₂OH, c-C₃H₅, cC₆H₁₁, (CH₂)₃OM₂CH₃, CH₂CH=CH₂, C(CH₂CH₃)(CH₂OH)₂, CH₂C≡CH, CH₂CO₂CH₂CH₃, CH₂CH₂F, (CH₂)₃O(CH₂)₁₁CH₃, CH₂CH₂N(CH₃)₂, CH₂CH₂OCH₂CH₂NH₂, CH₂CF₃, NHCH₂CO₂CH₂CH₃, CH(CH₃)CO₂CH₃, C(CH₃)₂CH₂C(O)CH₃, CH(CO₂CH₂CH₃)₂, CH₂CH₃, CH(CH₂CH₂CH₃)CO₂CH₃, CH₂CH₂CH₂OCH₃, C(CH₃)₂CH₂C=CH, (CH₂)₄CH₃, CH(CH₂CH₂CH₃)₂, (CH₂)SCH₃,CH₂CH₂CO₂H, CH(CH(CH₃)₂)CO₂CH₃, OCH₂CO₂H, CH(CH(CH₃)₂)CH₂OH, CH(CH(CH₃)₂)CH₂OH, CH(CH₃)CH₂OH, CH(CH₃)CH₂OH, CH(CH₃)₂, (CH₂)CH(CH₃)₂, CH(CH₃)CH₂CH₃, CH₂CH(CH₃)OH, (CH₂)₃CH₃, (CH₂)₂OCH₂CH₃, 1-Adamantyl, (CH₂)₈CH₃, CH(CH₃)CH(CH₃)₂, (CH₂)₃NHCH₃, (CH₂)₂N(CH₂CH₃)₂, 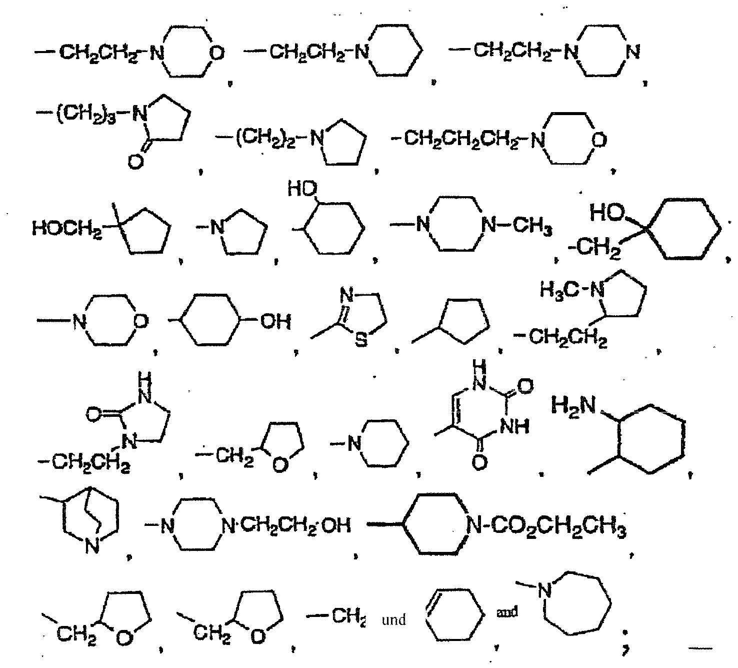
(2) ein pharmazeutisch oder tiermedizinisch verträgliches flüssiges Trägervehikel, umfassend ein Lösungsmittel und gegebenenfalls ein Colösungsmittel, wobei das Lösungsmittel ausgewählt ist aus der Gruppe bestehend aus Butyldiglycol, Dipropylenglycol-n-butylether, Diethylenglycolmonoethylether, Ethylenglycolmonomethylether, Dipropylenglycolmonomethylether, flüssigen Polyoxyethylenglycolen, Propylenglycol, Diethylenglycolmonoethylether, Ethylenglycol und einem Gemisch von mindestens zwei dieser Lösungsmittel, und das Colösungsmittel ausgewählt ist aus der Gruppe bestehend aus absolutem Ethanol, Isopropanol oder Methanol; und
(3) gegebenenfalls einen Kristallisationsinhibitor, ausgewählt aus der Gruppe bestehend aus einem anionischen grenzflächenaktiven Mittel, einem kationischen grenzflächenaktiven Mittel, einem nicht-ionischen grenzflächenaktiven Mittel, einem Aminsalz, einem amphoteren grenzflächenaktiven Mittel, Polyvinylpyrrolidon, Polyvinylalkoholen, Copolymeren von Vinylacetat und Vinylpyrrolidon, Polyethylenglycolen, Benzylalkohol, Mannit, Glycerin, Sorbitol, polyoxyethylenierten Sorbitanestern; Lecithin, Natriumcarboxymethylcellulose und Acrylderivaten oder einem Gemisch dieser Kristallisationsinhibitoren.

2. Die Spot-on-Formulierung nach Anspruch 1, wobei R^{x} C(CH₃)₃ ist.

3. Die Spot-on-Formulierung nach Anspruch 1, wobei das flüssige Trägervehikel eine Mikroemulsion umfasst.

4. Die Spot-on-Formulierung nach Anspruch 1, wobei das flüssige Trägervehikel weiter einen Exzipienten umfasst.

5. Die Spot-on-Formulierung nach Anspruch 4, wobei der Exzipient C₈-C₁₀-Capryl-/Caprintriglyceride, Ölsäure oder Propylenglycol ist.

6. Die Spot-on-Formulierung nach Anspruch 5, wobei die Spot-on-Formulierung weiter ein Antioxidationsmittel umfasst.

7. Die Spot-on-Formulierung nach Anspruch 6, wobei das Antioxidationsmittel aus der Gruppe bestehend aus butyliertem Hydroxyanisol, butyliertem Hydroxytoluol, Ascorbinsäure, Natriummetabisulfit, Propylgallat und Natriumthiosulfat ausgewählt ist.

8. Die Spot-on-Formulierung nach Anspruch 7, wobei die Verbindung der Formel (I) t-Butylnodulisporamid ist, das Trägermedium Diethylenglycolmonoethylether und C₈-C₁₀-Capryl-/Caprintriglyceride umfasst und das Antioxidationsmittel butyliertes Hydroxytoluol ist.

9. Die Spot-on-Formulierung nach Anspruch 1, wobei die Kombination etwa 0,001 bis etwa 100 mg/kg Gewicht des Säugers oder Vogels einer Verbindung der Formel (I) umfasst.

10. Die Spot-on-Formulierung nach Anspruch 2, wobei die Kombination etwa 1 bis etwa 50 mg/kg Gewicht des Säugers oder Vogels einer Verbindung der Formel (I) umfasst.

11. Die Spot-on-Formulierung nach Anspruch 1, welche einen Kristallisationsinhibitor umfasst und weiter ein Antioxidationsmittel umfasst.

12. Die Spot-on-Formulierung nach Anspruch 11, wobei etwa 0,005 bis etwa 1% (Gew./Vol.) eines Antioxidationsmittels vorhanden sind und das Antioxidationsmittel aus der Gruppe bestehend aus butyliertem Hydroxyanisol, butyliertem Hydroxytoluol, Ascorbinsäure, Natriummetabisulfit, Propylgallat und Natriumthiosulfat ausgewählt ist.

13. Die Spot-on-Formulierung nach Anspruch 12, wobei der Kristallisationsinhibitor in einer Menge von etwa 1 bis etwa 20% Gew./Vol. vorliegt.

14. Die Spot-on-Formulierung nach Anspruch 13, wobei
- das anionische grenzflächenaktive Mittel alkalische Stearate, Natriumabietat; Alkylsulfate; Natriumdodecylbenzolsulfonat, Natriumdioctylsulfosuccinat; und Fettsäuren ist;
- das kationische grenzflächenaktive Mittel wasserlösliche quartäre Ammoniumsalze der Formel N⁺R'R"R"'R""Y⁻ ist, wobei die Reste R unabhängig Kohlenwasserstoffreste, gegebenenfalls hydroxyliert, sind und Y⁻ ein Anion einer starken Säure ist;
- das Aminsalz ein Aminsalz von N⁺R'R"R"' ist, wobei die Reste R unabhängig gegebenenfalls hydroxylierte Kohlenwasserstoffreste sind;
- das nicht-ionische grenzflächenaktive Mittel gegebenenfalls polyoxyethylenierte Sorbitanester, polyoxyethylenierte Alkylether; Polyethylenglycolstearat, polyoxyethylenierte Derivate von Rhizinusöl, Polyglycerinester, polyoxyethylenierte Fettalkohole, polyoxyethylenierte Fettsäuren, Copolymere von Ethylenoxid und Propylenoxid ist; und
- das amphotere grenzflächenaktive Mittel Lauryl-substituierte Betainverbindungen ist.

15. Die Spot-on-Formulierung nach Anspruch 13, wobei der Kristallisationsinhibitor ein Kristallisationsinhibitorsystem ist, das ein polymeres filmbildendes Mittel und ein grenzflächenaktives Mittel umfasst.

16. Die Spot-on-Formulierung nach Anspruch 15, wobei das polymere filmbildende Mittel Polyvinylpyrrolidon, Polyvinylalkohole oder ein Copolymer von Vinylacetat und Polyvinylpyrrolidon ist und das grenzflächenaktive Mittel ein nicht-ionisches grenzflächenaktives Mittel ist.

17. Die Spot-on-Formulierung nach Anspruch 16, wobei das Kristallisationsinhibitorsystem ein Gemisch von Polyvinylpyrrolidon und Polyoxyethylen-(20)-sorbitanmonooleat ist.

18. Die Spot-on-Formulierung nach Anspruch 12, wobei die Verbindung der Formel (I) t-Butylnodulisporamid ist, das flüssige Trägervehikel Diethylenglycolmonoethylether ist, der Kristallisationsinhibitor Pyrrolidon ist und das Antioxidationsmittel butyliertes Hydroxytoluol ist.

19. Eine Spot-on-Formulierung zur Verwendung nach Anspruch 1, wobei der Parasit ein Ektoparasit ist.

20. Eine Spot-on-Formulierung zur Verwendung nach Anspruch 1, wobei der Parasit ein Endoparasit ist.

21. Eine Spot-on-Formulierung zur Verwendung nach Anspruch 1, wobei der Säuger eine Katze, ein Hund, ein Pferd, ein Rind oder ein Schaf ist.

22. Eine Spot-on-Formulierung zur Verwendung nach Anspruch 21, wobei der Parasit ein Floh oder eine Zecke ist.

23. Eine Spot-on-Formulierung zur Verwendung nach Anspruch 1, wobei der Säuger ein Mensch ist.

24. Eine Spot-on-Formulierung zur Verwendung nach Anspruch 19, wobei die Ektoparasiten Milben, Zecken, Mücken, Fliegen oder ein Kombination der vorstehenden sind.

25. Die Spot-on-Formulierung zur Verwendung nach Anspruch 1, wobei die Verabreichung zweimonatlich erfolgt.

26. Die Spot-on-Formulierung zur Verwendung nach Anspruch 1, wobei die Verabreichung vierteljährlich erfolgt.

27. Die Spot-on-Formulierung zur Verwendung nach Anspruch 1, wobei die Verabreichung monatlich erfolgt.

28. Eine Spot-on-Formulierung zur Verwendung nach Anspruch 1, welche für die lokalisierte kutane Anwendung auf einen Säuger mit Absorption und einer resultierenden Plasmakonzentration der Verbindung(en) der Formel (I) geeignet ist, wobei das flüssige Trägervehikel Diethylenglycolmonoethylether und mindestens ein Antioxidationsmittel umfasst.

29. Eine Spot-on-Formulierung zur Verwendung nach Anspruch 1, wobei der Säuger eine Katze oder ein Hund ist und wobei die Formulierung zur lokalisierten kutanen Anwendung auf die Katze oder den Hund zwischen den Schultern mit einer Häufigkeit von nicht mehr als einmal pro Monat geeignet ist, wobei die Formulierung ein organisches Lösungsmittel, ein Antioxidationsmittel und/oder einen Kristallisationsinhibitor enthält, wobei:
das organische Lösungsmittel Diethylglycolmonoethylether umfasst; wobei das Lösungsmittel gegebenenfalls mit C₈-C₁₀-Capryl-/Caprintriglycerid, Ölsäure oder Propylenglycol ergänzt ist;
das Antioxidationsmittel aus der Gruppe bestehend aus butyliertem Hydroxyanisol, butyliertem Hydroxytoluol, Ascorbinsäure, Natriummetabisulfit, Propylgallat und Natriumthiosulfat ausgewählt ist; und
der Kristallisationsinhibitor aus der Gruppe bestehend aus Polyvinylpyrrolidon, Copolymeren von Vinylacetat und Vinylpyrrolidon, polyoxyethylenierten Sorbitanestem und Gemischen davon ausgewählt ist;
wobei eine verlängerte Freisetzung der Formel (I) in oder auf dem Körper der Katze oder des Hundes stattfindet.

## Revendications

1. Formulation de dépôt destinée à être utilisée dans le traitement ou la prophylaxie d'une infestation par des parasites chez des mammifères, des poissons ou des oiseaux qui comprend
(1) une quantité efficace d'au moins un dérivé d'acide nodulisporique de formule (I) 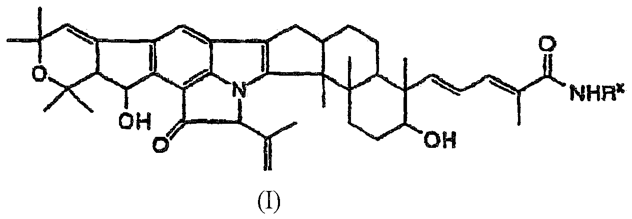 où R^{x} est choisi dans le groupe consistant en :
H, CH₃, CH₂CH₃, C(CH₃)₃, CH₂CH₂CH₃, CH₂CH₂OH, CH(CO₂CH₃)CH₂OH, CH₂CO₂CH₃, CH₂CH(OCH₂CH₃)₂, CH₂CH₂OCH₂CH₂OH, CH(CH₃)(CH₂)₃C(CH₃)₂OH, (CH₂)₃OH, (CH₂)₄OH, (CH₂)SOH, CH(CH₂OH)CH₂CH₃, NHC(CH₃)₃, CH₂CN, (CH₂)₆OH, CH₂CH(OH)CH₃, CH(CH₂OH)CH₂CH₂CH₃, CH₂CH₂SCH₃, CH₂CH₂SCH₂CH₃, CH₂CONH₂, CH(CH₃)(CH₂OH)₂, CH₂CH₂NHCH₂CH₂OH, CH(CH₂OH)(CH₂)₃CH₃, CH(CH₂OCH₃)CH₃, (CH₂)₂SH, (CH₂)₄NH₂, CH₂CH₂SO₂CH₃, CH₂CH₂S(O)CH₃, CH(CH(CH₃)₂)CH₂OH, (CH₂)₃NH₂, (CH₂)₃N(CH₂CH₃)₂, (CH₂)₃N(CH₃)₂, OCH₂CH₃, CH₂CH(OH)CH₂OH, OCH₃, CH₂CH₂OCH₃, CH₂CH₂NHC(O)CH₃, C(CH₃)₂CH₂OH, c-C₃H₅, cC₆H₁₁, (CH₂)₃OCH₂CH₃, CH₂CH=CH₂, C(CH₂CH₃)(CH₂OH)₂, CH₂C=CH, CH₂CO₂CH₂CH₃, CH₂CH₂F, (CH₂)₃O(CH₂₎₁₁CH₃, CH₂CH₂N(CH₃)₂, CH₂CH₂OCH₂CH₂NH₂, CH₂CF₃, NHCH₂CO₂CH₂CH₃, CH(CH₃)CO₂CH₃, C(CH₃)₂CH₂C(O)CH₃, CH(CO₂CH₂CH₃)₂, CH₂CH₃, CH(CH₂CH₂CH₃)CO₂CH₃, CH₂CH₂CH₂OCH₃, C(CH₃)₂CH₂C≡CH, (CH₂)₄CH₃, CH(CH₂CH₂CH₃)₂, (CH₂)SCH₃, CH₂CH₂CO₂H, CH(CH(CH₃)₂)CO₂CH₃, OCH₂CO₂H, CH(CH(CH₃)₂)CH₂OH, CH(CH(CH₃)₂)CH₂OH, CH(CH₃)CH₂OH, CH(CH₃)CH₂OH, CH(CH₃)₂, (CH₂)CH(CH₃)₂, CH(CH₃)CH₂CH₃, CH₂CH(CH₃)OH, (CH₂)₃CH₃, (CH₂)₂OCH₂CH₃, 1-adamantyle, (CH₂)₈CH₃, CH(CH₃)CH(CH₃)₂, (CH₂)₃NHCH₃, (CH₂)₂N(CH₂CH₃)₂, 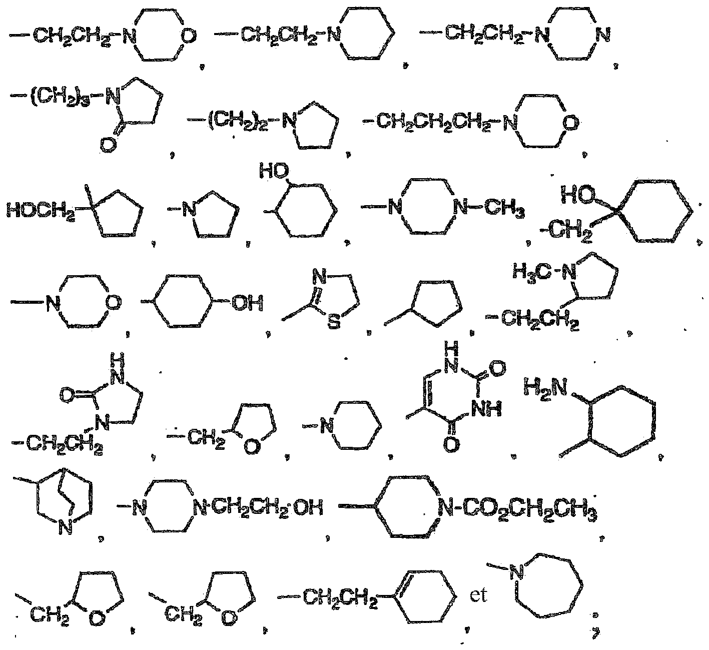
(2) un véhicule vecteur liquide acceptable du point de vue pharmaceutique ou vétérinaire comprenant un solvant et éventuellement un cosolvant où le solvant est choisi dans le groupe consistant en le butyldiglycol, le n-butyléther de dipropylèneglycol, le monoéthyléther de diéthylèneglycol, le monométhyléther d'éthylèneglycol, le monométhyléther de dipropylèneglycol, les polyoxyéthylèneglycols liquides, le propylèneglycol, le monoéthyléther de diéthylèneglycol, l'éthylèneglycol, et un mélange d'au moins deux de ces solvants et le cosolvant est choisi dans le groupe consistant en l'éthanol, l'isopropanol ou le méthanol absolu ; et
(3) éventuellement un inhibiteur de cristallisation choisi dans le groupe consistant en un tensioactif anionique, un tensioactif cationique, un tensioactif non ionique, un sel d'amine, un tensioactif amphotère, la polyvinylpyrrolidone, les poly(alcools vinyliques), les copolymères d'acétate de vinyle et de vinylpyrrolidone, les polyéthylèneglycols, l'alcool benzylique, le mannitol, le glycérol, le sorbitol, les esters de sorbitan polyoxyéthylénés ; la lécithine, la carboxyméthylcellulose sodique et les dérivés acryliques, ou un mélange de ces inhibiteurs de cristallisation.

2. Formulation de dépôt selon la revendication 1 où R^{x} est C(CH₃)₃.

3. Formulation de dépôt selon la revendication 1 où le véhicule vecteur liquide comprend une microémulsion.

4. Formulation de dépôt selon la revendication 1 où le véhicule vecteur liquide comprend en outre un excipient.

5. Formulation de dépôt selon la revendication 4 où l'excipient est des triglycérides C₈-C₁₀ capryliques/capriques, l'acide oléique ou le propylèneglycol.

6. Formulation de dépôt selon la revendication 5 où la formulation de dépôt comprend en outre un antioxydant.

7. Formulation de dépôt selon la revendication 6 où l'antioxydant est choisi dans le groupe consistant en l'hydroxyanisole butylé, l'hydroxytoluène butylé, l'acide ascorbique, le métabisulfite de sodium, le gallate de propyle et le thiosulfate de sodium.

8. Formulation de dépôt selon la revendication 7 où le composé de formule (I) est le nodulisporamide de t-butyle, le milieu vecteur comprend du monoéthyléther de diéthylèneglycol et des triglycérides C₈-C₁₀ capryliques/ capriques et l'antioxydant est l'hydroxytoluène butylé.

9. Formulation de dépôt selon la revendication 1 où la combinaison comprend environ 0,001 à environ 100 mg/kg de poids de mammifère ou d'oiseau d'un composé de formule (I).

10. Formulation de dépôt selon la revendication 2 où la combinaison comprend environ 1 à environ 50 mg/kg de poids de mammifère ou d'oiseau d'un composé de formule (I).

11. Formulation de dépôt selon la revendication 1 qui comprend un inhibiteur de cristallisation et comprend en outre un antioxydant.

12. Formulation de dépôt selon la revendication 11 où environ 0,005 à environ 1 % (P/V) d'antioxydant est présent et l'antioxydant est choisi dans le groupe consistant en l'hydroxyanisole butylé, l'hydroxytoluène butylé, l'acide ascorbique, le métabisulfite de sodium, le gallate de propyle et le thiosulfate de sodium.

13. Formulation de dépôt selon la revendication 12 où l'inhibiteur de cristallisation est présent en une quantité d'environ 1 à environ 20 % P/V.

14. Formulation de dépôt selon la revendication 13 où
- le tensioactif anionique est des stéarates alcalins, l'abiétate de sodium ; des sulfates d'alkyle ; le dodécylbenzènesulfonate de sodium, le dioctylsulfosuccinate de sodium ; et des acides gras ;
- le tensioactif cationique est des sels d'ammonium quaternaire solubles dans l'eau de formule N⁺R'R"R"'R""Y⁻ où les radicaux R sont indépendamment des radicaux hydrocarbonés, éventuellement hydroxylés, et Y⁻ est un anion d'un acide fort ;
- le sel d'amine est un sel d'amine de N⁺R'R"R"' où les radicaux R sont indépendamment des radicaux hydrocarbonés éventuellement hydroxylés ;
- le tensioactif non ionique est des esters de sorbitan éventuellement polyoxyéthylénés, des alkyléthers éventuellement polyoxyéthylénés ; le stéarate de polyéthylèneglycol, des dérivés polyoxyéthylénés d'huile de ricin, des esters de polyglycérol, des alcools gras polyoxyéthylénés, des acides gras polyoxyéthylénés, des copolymères d'oxyde d'éthylène et d'oxyde de propylène ; et
- le tensioactif amphotère est des composés de bétaïne lauryl-substitués.

15. Formulation de dépôt selon la revendication 13 où l'inhibiteur de cristallisation est un système d'inhibiteur de cristallisation comprenant un agent filmogène polymérique et un tensioactif.

16. Formulation de dépôt selon la revendication 15 où l'agent filmogène polymérique est la polyvinylpyrrolidone, des poly(alcools vinyliques), ou un copolymère d'acétate de vinyle et de polyvinylpyrrolidone et le tensioactif est un tensioactif non ionique.

17. Formulation de dépôt selon la revendication 16 où le système d'inhibiteur de cristallisation est un mélange de polyvinylpyrrolidone et de mono-oléate de polyoxyéthylène (20) sorbitan.

18. Formulation de dépôt selon la revendication 12 où le composé de formule (I) est le nodulisporamide de t-butyle, le véhicule vecteur liquide est le monoéthyléther de diéthylèneglycol, l'inhibiteur de cristallisation est la pyrrolidone et l'antioxydant est l'hydroxytoluène butylé.

19. Formulation de dépôt destinée à être utilisée selon la revendication 1 où le parasite est un ectoparasite.

20. Formulation de dépôt destinée à être utilisée selon la revendication 1 où le parasite est un endoparasite.

21. Formulation de dépôt destinée à être utilisée selon la revendication 1 où le mammifère est un chat, un chien, un cheval, du bétail ou un mouton.

22. Formulation de dépôt destinée à être utilisée selon la revendication 21 où le parasite est une puce ou une tique.

23. Formulation de dépôt destinée à être utilisée selon la revendication 1 où le mammifère est un humain.

24. Formulation de dépôt destinée à être utilisée selon la revendication 19 où les ectoparasites sont des acariens, des tiques, des moustiques, des mouches ou une combinaison des précédents.

25. Formulation de dépôt destinée à être utilisée selon la revendication 1 où l'administration est bimensuelle.

26. Formulation de dépôt destinée à être utilisée selon la revendication 1 où l'administration est trimestrielle.

27. Formulation de dépôt destinée à être utilisée selon la revendication 1 où l'administration est mensuelle.

28. Formulation de dépôt destinée à être utilisée selon la revendication 1 qui est appropriée pour l'application cutanée localisée à un mammifère avec absorption et une concentration plasmatique résultante du ou des composés de formule (I) où le véhicule vecteur liquide comprend du monoéthyléther de diéthylèneglycol, et au moins un antioxydant.

29. Formulation de dépôt destinée à être utilisée selon la revendication 1 où le mammifère est un chat ou un chien et où ladite formulation est appropriée pour l'application cutanée localisée au chat ou au chien, entre les épaules, à une fréquence ne dépassant pas mensuellement, où la formulation contient un solvant organique, un antioxydant et/ou un inhibiteur de cristallisation où :
le solvant organique comprend du monoéthyléther de diéthylglycol ; ledit solvant éventuellement additionné de triglycéride C₈-C₁₀ caprylique/caprique, d'acide oléique ou de propylèneglycol ;
l'antioxydant est choisi dans le groupe consistant en l'hydroxyanisole butylé, l'hydroxytoluène butylé, l'acide ascorbique, le métabisulfite de sodium, le gallate de propyle et le thiosulfate de sodium ; et
l'inhibiteur de cristallisation choisi dans le groupe consistant en la polyvinylpyrrolidone, les copolymères d'acétate de vinyle et de vinylpyrrolidone, les esters de sorbitan polyoxyéthylénés et leurs mélanges ;
de sorte qu'il y a une libération prolongée de la formule (I) dans ou sur le corps du chat ou du chien.
